(19) **Europäisches Patentamt / European Patent Office / Office européen des brevets**

(11) **EP 3 527 124 B1**

(12) # EUROPEAN PATENT SPECIFICATION

(45) Date of publication and mention
of the grant of the patent:
**23.11.2022 Bulletin 2022/47**

(21) Application number: **19156515.9**

(22) Date of filing: **11.02.2019**

(51) International Patent Classification (IPC):
**A61B 5/00** *(2006.01)*     **G06F 21/32** *(2013.01)*
**G06K 9/00** *(2022.01)*     **A61B 5/145** *(2006.01)*

(52) Cooperative Patent Classification (CPC):
**A61B 5/14532; A61B 5/443; A61B 5/4872;**
**A61B 5/4875; A61B 5/6898; G06F 21/32;**
A61B 5/0004; A61B 5/6803; A61B 5/681;
A61B 5/7235; A61B 5/7275; A61B 5/7405;
A61B 5/7475; A61B 2562/043; A61B 2562/16

(54) **ELECTRONIC DEVICE INCLUDING SENSOR FOR SENSING BIOMETRIC INFORMATION USING MULTIPLE RECEIVING UNITS**

ELEKTRONISCHE VORRICHTUNG MIT SENSOR ZUR ERFASSUNG BIOMETRISCHER INFORMATIONEN MITHILFE MEHRERER EMPFANGSEINHEITEN

DISPOSITIF ÉLECTRONIQUE COMPRENANT UN CAPTEUR POUR DÉTECTER DES INFORMATIONS BIOMÉTRIQUES UTILISANT DE MULTIPLES UNITÉS DE RÉCEPTION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **14.02.2018 KR 20180018695**

(43) Date of publication of application:
**21.08.2019 Bulletin 2019/34**

(73) Proprietor: **Samsung Electronics Co., Ltd.**
**Suwon-si, Gyeonggi-do, 16677 (KR)**

(72) Inventors:
• **CHUNG, Sohyun**
  **16677 Gyeonggi-do (KR)**
• **LEE, Yongjin**
  **16677 Gyeonggi-do (KR)**
• **LEE, Hongji**
  **16677 Gyeonggi-do (KR)**
• **KIM, Minji**
  **16677 Gyeonggi-do (KR)**

• **KIM, Taeho**
  **16677 Gyeonggi-do (KR)**
• **MUN, Gitae**
  **16677 Gyeonggi-do (KR)**
• **PARK, Jongin**
  **16677 Gyeonggi-do (KR)**
• **LEE, Seunggoo**
  **16677 Gyeonggi-do (KR)**
• **LEE, Wonseok**
  **16677 Gyeonggi-do (KR)**

(74) Representative: **Arnold & Siedsma**
**Bezuidenhoutseweg 57**
**2594 AC The Hague (NL)**

(56) References cited:
EP-A2- 3 120 770     WO-A1-2017/027551
WO-A1-2017/197033     US-A1- 2008 108 887
US-A1- 2012 148 143     US-A1- 2016 061 726

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

**Description**

BACKGROUND

1. Field

The present disclosure relates to electronic devices including a sensor for sensing biometric information using a plurality of receiving units, and methods for controlling the same.

2. Description of Related Art

The recent spread of smartphones and other electronic devices has lead to an increasing demand for users to receive various pieces of information on their electronic device. In response, manufacturers are coming up with electronic devices that pack a biometric sensor with various functionalities, e.g., electronic devices capable of obtaining biometric information, such as a heartbeat or oxygen saturation, or electronic devices with other various capabilities.

Such electronic devices may obtain various pieces of biometric information, e.g., a user's heartbeat, oxygen saturation, stress level, or blood pressure, by using a biometric sensor. For example, the electronic device may sense a part of the user's body using the biometric sensor in response to the user's request. The electronic device may acquire pieces of the user's biometric information by using the information obtained by the sensor.

The biometric sensor-equipped electronic device, e.g., a smartphone, may only provide the user with limited biometric information, e.g., heartbeat or oxygen saturation, using the biometric sensor, but not other pieces of information that the user may be more interested in, such as glucose, water, or fat amount, in their daily life.

The biometric sensor-equipped electronic device may merely deliver the user's biometric information obtained by the biometric sensor to the user but cannot compare or analyze the pieces of biometric information obtained.

WO-2017/027551 discloses a device for monitoring / gathering biometric information

SUMMARY

The invention is defined by a wearable electronic device according to claim **1**. Embodiments of the invention are defined by dependent claims **2-3**.

BRIEF DESCRIPTION OF THE DRAWINGS

The above and other aspects, features, and advantages of certain embodiments of the present disclosure will be more apparent from the following description, taken in conjunction with the accompanying drawings, in which:

FIG. 1 is a block diagram of an electronic device in a network environment, according to an embodiment;

FIG. 2A is a block diagram of a sensor module, according to an embodiment;

FIG. 2B is an illustration of an electronic device with a sensor module, according to an embodiment;

FIGs. 3A, 3B, 3C, 3D, and 3E are illustrations of a sensor module, according to an embodiment; the embodiment of FIG. 3C not falling under the scope of the claims;

FIGs. 4A and 4B are block diagrams illustrating pluses of light modulated as per frequency, which are output from an electronic device, according to an embodiment;

FIGs. 5A and 5B are block diagrams illustrating pulses of light modified in height and/or width, which are output from an electronic device, according to an embodiment;

FIG. 5C is a block diagram illustrating pulses of light with same amplitude or strength, which are output from an electronic device, according to an embodiment;

FIGs. 6A, 6B, and 6C are a flowchart, a graph, and an illustration of an electronic device's function/operation for providing biometric information based on variations in amplitude and phase, the function/operation and associated methods not falling under the scope of the claims by themselves;

FIGs. 7A, 7B, and 7C are a flowchart and illustrations of an electronic device's function/operation for providing comparison data regarding biometric information, the function/operation and associated methods not falling under the scope of the claims by themselves;

FIGs. 8A and 8B are flowcharts of an electronic device's function/operation for obtaining biometric information using an absorption coefficient and a scattering coefficient and providing the obtained biometric information, the function/ operation and associated methods not falling under the scope of the claims by themselves;

FIGs. 9A, 9B, and 9C are illustrations of an electronic device's function/operation for obtaining the amount of body fat and providing information about the obtained body fat amount, the function/operation and associated methods not falling under the scope of the claims by themselves;

FIGs. 9D, 9E, 9F, 9G, and 9H are illustrations of an electronic device's function/operation for obtaining an amount of glucose, or its variation, in blood, and providing information about the obtained glucose amount or variation, the function/ operation and associated methods not falling under the scope of the claims by themselves;

FIGs. 10A and 10B are illustrations of an electronic device's function/operation for obtaining an amount of water and providing information about the obtained water amount, the function/operation and associated methods not falling under the scope of the claims by themselves;

FIGs. 11A and 11B are illustrations of a function/operation for providing comprehensive biometric information based on biometric information, the function/ operation and associated methods not falling under the scope of the claims by themselves;

FIGs. 12A and 12B are block diagrams of an electronic device's function/operation for outputting light based on the frequency varied by the skin tone automatically detected, the function/operation and associated methods not falling under the scope of the claims by themselves;

FIGs. 13A, 13B, and 13C are a flowchart and illustrations of an electronic device's function/operation for comparing a variation rate of reference biometric information and a variation rate of target biometric information and providing the results, the function/operation and associated methods not falling under the scope of the claims by themselves;

FIGs. 14A and 14B are views illustrating an electronic device's function/operation for separately providing a fat quantity in blood and a fat quantity in tissue, the function/operation and associated methods not falling under the scope of the claims by themselves;

FIG. 15 is a graph of an absorption spectrum for a body composition,

FIG. 16 is an illustration of a function/operation for providing a user with biometric information obtained by an electronic device via a sound output device, the function/operation and associated methods not falling under the scope of the claims by themselves; and

FIGs. 17A and 17B are flowcharts of a method for operating an electronic device, the method not falling under the scope of the claims.

## DETAILED DESCRIPTION

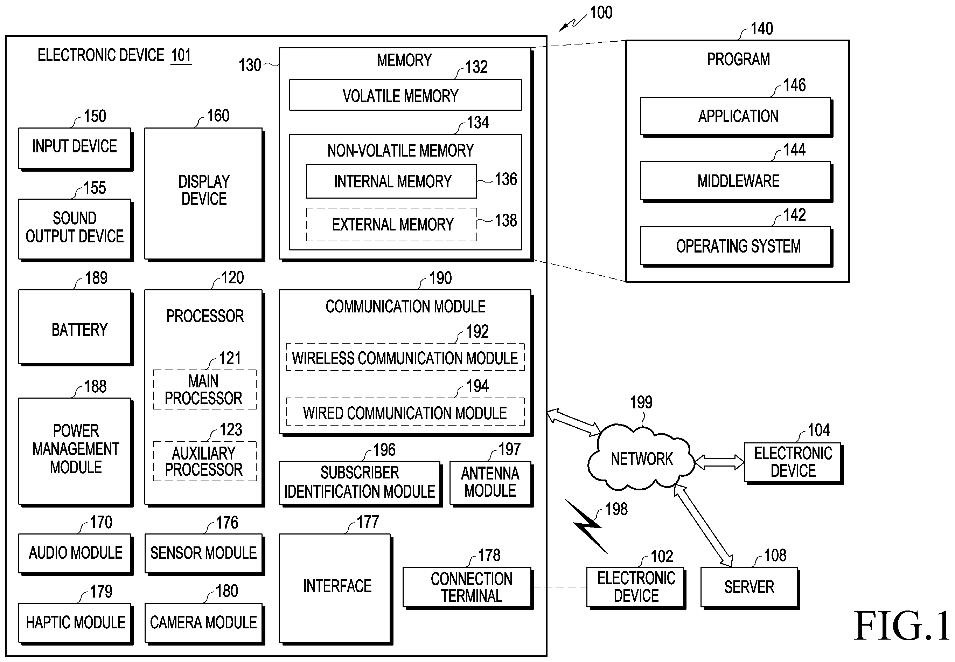

FIG. 1 is a block diagram of an electronic device 101 in a network environment 100 according to various embodiments.

Referring to FIG. 1, the electronic device 101 in the network environment 100 may communicate with an electronic device 102 via a first network 198 (e.g., a short-range wireless communication network), or an electronic device 104 or a server 108 via a second network 199 (e.g., a long-range wireless communication network). According to an embodiment, the electronic device 101 may communicate with the electronic device 104 via the server 108. According to an embodiment, the electronic device 101 may include a processor 120, memory 130, an input device 150, a sound output device 155, a display device 160, an audio module 170, a sensor module 176, an interface 177, a haptic module 179, a camera module 180, a power management module 188, a battery 189, a communication module 190, a subscriber identification module (SIM) 196, or an antenna module 197. In some embodiments, at least one (e.g., the display device 160 or the camera module 180) of the components may be omitted from the electronic device 101, or one or more other components may be added in the electronic device 101. In some embodiments, some of the components may be implemented as single integrated circuitry. For example, the sensor module 176 (e.g., a fingerprint sensor, an iris sensor, or an illuminance sensor) may be implemented as embedded in the display device 160 (e.g., a display).

The processor 120 may execute, e.g., software (e.g., a program 140) to control at least one other component (e.g., a hardware or software component) of the electronic device 101 connected with the processor 120 and may process or compute various data. According to one embodiment, as at least part of the data processing or computation, the processor 120 may load a command or data received from another component (e.g., the sensor module 176 or the communication module 190) in volatile memory 132, process the command or the data stored in the volatile memory 132, and store resulting data in non-volatile memory 134. According to an embodiment, the processor 120 may include a main processor 121 (e.g., a central processing unit (CPU) or an application processor (AP)), and an auxiliary processor 123 (e.g., a graphics processing unit (GPU), an image signal processor (ISP), a sensor hub processor, or a communication processor (CP)) that is operable independently from, or in conjunction with, the main processor 121. Additionally or alternatively, the auxiliary processor 123 may be adapted to consume less power than the main processor 121, or to be specific to a specified function. The auxiliary processor 123 may be implemented as separate from, or as part of the main processor 121.

The auxiliary processor 123 may control at least some of functions or states related to at least one (e.g., the display device 160, the sensor module 176, or the communication module 190) of the components of the electronic device 101, instead of the main processor 121 while the main processor 121 is in an inactive (e.g., sleep) state or along with the main processor 121 while the main processor 121 is an active state (e.g., executing an application). According to an embodiment, the auxiliary processor 123 (e.g., an image signal processor or a communication processor) may be implemented as part of another component (e.g., the camera module 180 or the communication module 190) functionally related to the auxiliary processor 123.

The memory 130 may store various data used by at least one component (e.g., the processor 120 or the sensor module 176) of the electronic device 101. The various data may include, for example, software (e.g., the program 140) and input data or output data for a command related thererto. The memory 130 may include the volatile memory 132 or the non-volatile memory 134.

The program 140 may be stored in the memory 130 as software, and may include, for example, an operating system (OS) 142, middleware 144, or an application 146.

The input device 150 may receive a command or data to be used by other component (e.g., the processor 120) of the electronic device 101, from the outside (e.g., a user) of the electronic device 101. The input device 150 may include, for example, a microphone, a mouse, or a keyboard.

The sound output device 155 may output sound signals to the outside of the electronic device 101. The sound output device 155 may include, for example, a speaker or a receiver. The speaker may be used for general purposes, such as playing multimedia or playing record, and the receiver may be used for an incoming calls. According to an embodiment, the receiver may be implemented as separate from, or as part of the speaker.

The display device 160 provides information to the outside (e.g., a user) of the electronic device 101. The display device 160 may include, for example, a display, a hologram device, or a projector and control circuitry to control a corresponding one of the display, hologram device, and projector. According to an embodiment, the display device 160 may include touch circuitry adapted to detect a touch, or sensor circuitry (e.g., a pressure sensor) adapted to measure the intensity of force incurred by the touch.

The audio module 170 may convert a sound into an electrical signal and vice versa. According to an embodiment, the audio module 170 may obtain a sound through the input device 150 or output a sound through the sound output device 155 or an external electronic device (e.g., an electronic device 102 (e.g., a speaker or a headphone) directly or wirelessly connected with the electronic device 101.

The sensor module 176 may detect an operational state (e.g., power or temperature) of the electronic device 101 or an environmental state (e.g., a state of a user) external to the electronic device 101, and then generate an electrical signal or data value corresponding to the detected state. According to an embodiment, the sensor module 176 may include, for example, a gesture sensor, a gyro sensor, an atmospheric pressure sensor, a magnetic sensor, an acceleration sensor, a grip sensor, a proximity sensor, a color sensor, an infrared (IR) sensor, a biometric sensor, a temperature sensor, a humidity sensor, or an illuminance sensor.

The interface 177 may support one or more specified protocols to be used for the electronic device 101 to be coupled with the external electronic device (e.g., the electronic device 102) directly (e.g., wiredly) or wirelessly. According to an embodiment, the interface 177 may include, for example, a high definition multimedia interface (HDMI), a universal serial bus (USB) interface, a secure digital (SD) card interface, or an audio interface.

A connecting terminal 178 may include a connector via which the electronic device 101 may be physically connected with the external electronic device (e.g., the electronic device 102). According to an embodiment, the connecting terminal 178 may include, for example, a HDMI connector, a USB connector, a SD card connector, or an audio connector (e.g., a headphone connector).

The haptic module 179 may convert an electrical signal into a mechanical stimulus (e.g., a vibration or motion) or electrical stimulus which may be recognized by a user via his tactile sensation or kinesthetic sensation. According to an embodiment, the haptic module 179 may include, for example, a motor, a piezoelectric element, or an electric stimulator.

The camera module 180 may capture a still image or moving images. According to an embodiment, the camera module 180 may include one or more lenses, image sensors, image signal processors, or flashes.

The power management module 188 may manage power supplied to the electronic device 101. According to one embodiment, the power management module 188 may be implemented as at least part of, for example, a power management integrated circuit (PMIC).

The battery 189 may supply power to at least one component of the electronic device 101. According to an embodiment, the battery 189 may include, for example, a primary cell which is not rechargeable, a secondary cell which is rechargeable, or a fuel cell.

The communication module 190 may support establishing a direct (e.g., wired) communication channel or wireless communication channel between the electronic device 101 and an external electronic device (e.g., the electronic device 102, the electronic device 104, or the server 108) and performing communication through the established communication channel. The communication module 190 may include one or more communication processors that are operable independently from the processor 120 (e.g., the application processor (AP)) and supports a direct (e.g., wired) communication or a wireless communication. According to an embodiment, the communication module 190 may include a wireless communication module 192 (e.g., a cellular communication module, a short-range wireless communication module, or a global navigation satellite system (GNSS) communication module) or a wired communication module 194 (e.g., a local area network (LAN) communication module or a power line communication (PLC) module). A corresponding one of these communication modules may communicate with the external electronic device via the first network 198 (e.g., a short-range communication network, such as Bluetooth®, wireless-fidelity (Wi-Fi) direct, or a standard of the Infrared Data Association (IrDA)) or the second network 199 (e.g., a long-range communication network, such as a cellular network, the Internet, or a computer network (e.g., LAN or wide area network (WAN)). These various types of communication modules may be implemented as a single component (e.g., a single chip), or may be implemented as multi components (e.g., multi chips) separate from each other. The wireless communication module 192 may identify and authenticate the electronic device 101 in a communication network, such as the first network 198 or the second network 199, using subscriber information (e.g., international mobile subscriber identity (IMSI)) stored in the subscriber identification module 196.

The antenna module 197 may transmit or receive a signal or power to or from the outside (e.g., the external electronic device) of the electronic device 101. According to an embodiment, the antenna module 197 may include one or more antennas, and, therefrom, at least one antenna appropriate for a communication scheme used in the communication network, such as the first network 198 or the second network 199, may be selected, for example, by the communication module 190 (e.g., the wireless communication module 192). The signal or the power may then be transmitted or received between the communication module 190 and the external electronic device via the selected at least one antenna.

At least some of the above-described components may be coupled mutually and communicate signals (e.g., commands or data) therebetween via an inter-peripheral communication scheme (e.g., a bus, general purpose input and output (GPIO), serial peripheral interface (SPI), or mobile industry processor interface (MIPI)).

According to an embodiment, commands or data may be transmitted or received between the electronic device 101 and the external electronic device 104 via the server 108 coupled with the second network 199. The first and second external electronic devices 102 and 104 each may be a device of the same or a different type from the electronic device 101. According to an embodiment, all or some of operations to be executed at the electronic device 101 may be executed at one or more of the external electronic devices 102, 104, or 108. For example, if the electronic device 101 should perform a function or a service automatically, or in response to a request from a user or another device, the electronic device 101, instead of, or in addition to, executing the function or the service, may request the one or more external electronic devices to perform at least part of the function or the service. The one or more external electronic devices receiving the request may perform the at least part of the function or the service requested, or an additional function or an additional service related to the request, and transfer an outcome of the performing to the electronic device 101. The electronic device 101 may provide the outcome, with or without further processing of the outcome, as at least part of a reply to the request. To that end, a cloud computing, distributed computing, or client-server computing technology may be used, for example.

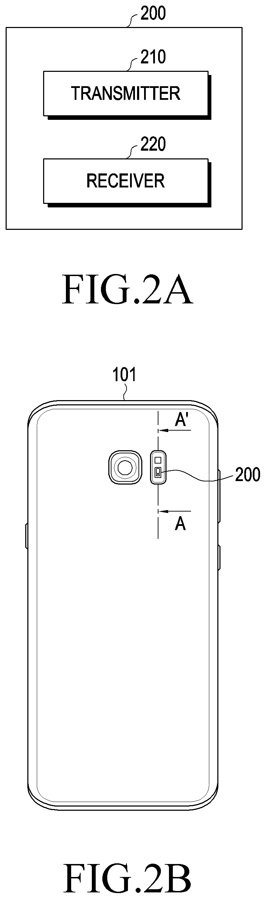

FIG. 2A is a block diagram illustrating a sensor module 200 according to an embodiment. FIG. 2B is an illustration of an electronic device with a sensor module according to an embodiment. The sensor module 200 includes at least one of an optical sensor module or an ultrasonic sensor module.

Referring to FIG. 2A, the sensor module 200 (e.g., an optical sensor module) includes a transmitter 210 and a receiver 220.

According to an embodiment, the transmitter 210 may include at least one light emitting device. The light emitting device (or light source) may include at least one of a vertical cavity surface emitting laser (VCSEL), a light emitting diode (LED), a white LED, a white laser, or a super continuum laser. However, this is merely an example, and the light emitting device may include various devices capable of emitting various wavelengths of light. The light emitting device may output at least one light beam to a user to obtain the user's biometric information.

According to an embodiment, when the sensor module 200 is implemented as an ultrasonic sensor module, the transmitter 210 may include at least one oscillation module that converts electrical signals into ultrasound waves and emits the ultrasound waves in the air. The oscillation module may include at least one of a magnet, a vibrating plate, or a piezoelectric material (or an element containing a piezoelectric material).

According to an embodiment, the receiver 220 may include at least one light receiving device. The light receiving device may include at least one of an avalanche photodiode (APD), a single photon avalanche diode (SPAD), a photodiode, a photomultiplier tube (PMT), a charge-coupled device (CCD), a complementary metal-oxide-semiconductor (CMOS) array, or a spectrometer. The light receiving device may include other various light receiving devices. The light receiving device may include silicon (Si) or indium gallium arsenide (InGaAs) depending on the wavelength of the light emitting device contained in the transmitter 210 (e.g., a light emitting device). The receiver 220 may include an arrayed light receiving device. When the receiver 220 (e.g., a light receiving device) includes a plurality of light receiving devices, some of the light receiving devices may be configured to directly receive light from the transmitter 210 directly, i.e., may receive light from the transmitter 210, which is not reflected by a user's body. The other light receiving devices may be configured to receive light that is emitted from the transmitter 210, strikes at least a part of a user's body, undergoes a light-tissue interaction, and is then reflected back to the sensor module 200.

According to an embodiment, when the sensor module 200 is implemented as an ultrasonic sensor module, the receiver 220 may include an ultrasonic scheme-based ultrasound wave receiving device configured to receive ultrasound waves and convert the ultrasound waves into electrical signals. For example, the ultrasound wave receiving device may include at least one of a magnet, a vibrating plate, or a piezoelectric material (or a device containing a piezoelectric material).

Referring to FIG. 2B, according to an embodiment, the sensor module 200 (e.g., an optical sensor module) may be disposed on a rear surface (e.g., a surface facing away from a surface where at least part of the display device 160 of FIG. 1 is exposed) of the electronic device 101. As shown in FIG. 2B, the transmitter 210 may be disposed to emit light to the outside of the electronic device 101. The receiver 220 may be disposed to receive light that is emitted from the transmitter 210, undergoes a tissue-light interaction, and is then reflected back to the sensor module 200 by at least a part of a user's body.

According to an embodiment, the sensor module 200 (e.g., an optical sensor module) may be disposed on a front surface (e.g., a surface facing away from the rear surface of the electronic device 101 of FIG. 1) of the electronic device 101. The electronic device 101 may include a wearable device (e.g., a smartwatch or earbuds). The sensor module 200 is disposed in at least a portion of the wearable device.

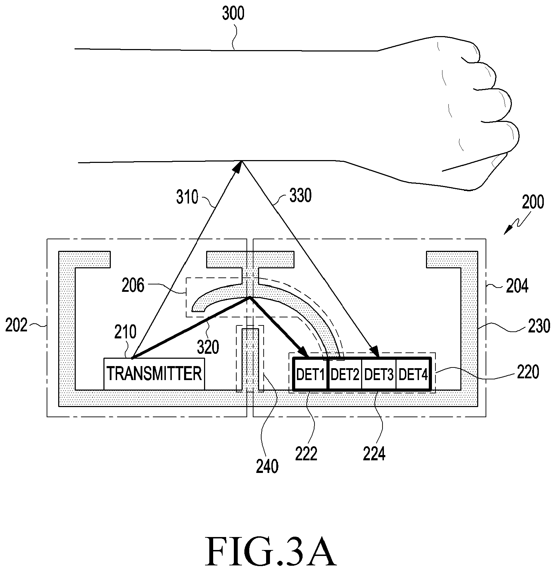

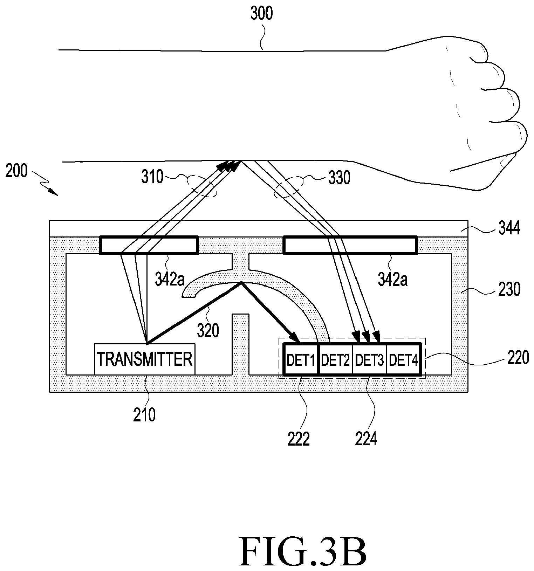

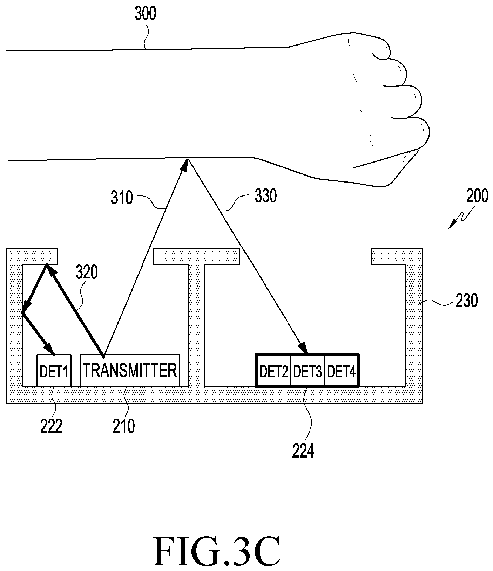

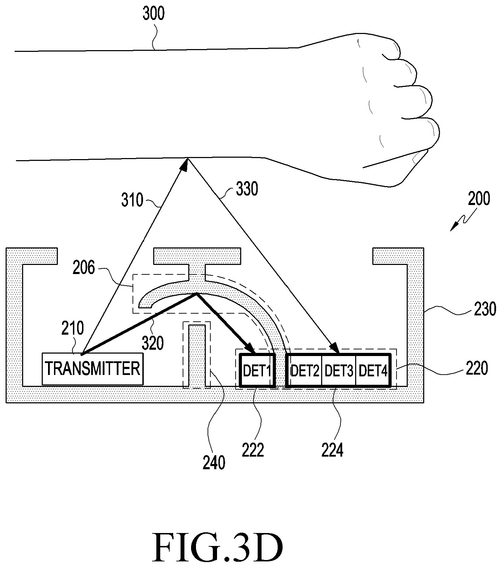

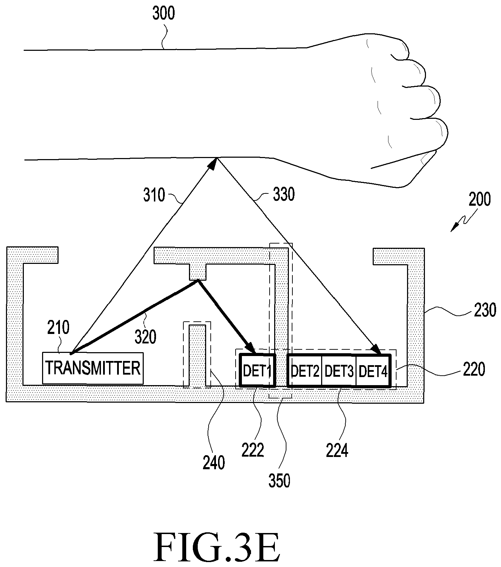

FIGs. 3A, 3B, 3C, 3D, and 3E are illustrations of a sensor module 200 (e.g., an optical sensor module) according to an embodiment. FIGs. 3A, 3B, 3C, 3D, and 3E are cross sections of the sensor module 200 according to an embodiment. The embodiment of fig. 3C does not fall under the scope of the appended claims.

Referring to FIG. 3A, the sensor module 200 includes a housing 230. According to the invention, the housing 230 may include a first wall 240 and a light path changer 206. The term "wall" may be interchangeably used with the term "rib." The term "light path changer 206" may be interchangeably used with the term "guide." The sensor module 200 includes a first area 202 and a second area 204 separated by the first wall 240. The light path changer 206 is disposed over the first area 202 and the second area 204 as shown in FIG. 3A. A portion of the light path changer 206 contacts any one light receiving device (e.g., a second light receiving device (detector (DET) 2)). The transmitter 210 (e.g., a light emitting device) is disposed in the first area 202 of the housing 230. The receiver 220 (e.g., a light receiving device) may be disposed in the first area 202 (which is not falling under the scope of the appended claims) or, according to the invention, the second area 204 of the housing 200. The receiver 220 includes a plurality of light receiving devices (e.g., a first light receiving device (DET 1), the second light receiving device (DET 2), a third light receiving device (DET 3), and a fourth light receiving device (DET 4)). The number (e.g., 4) of light receiving devices shown and described herein is merely an example to describe various embodiments of the present disclosure. Thus, according to an embodiment, the receiver 220 may include one (not falling under the scope of the claims) or, according to the invention, more light receiving devices or one (not falling under the scope of the appended claims) or, according to the invention, more ultrasound wave receiving devices.

According to an embodiment, the transmitter 210 may include at least one light emitting device or at least one ultrasonic oscillation device. The transmitter 210 may emit light to the outside (e.g., towards a user). As used herein, light emitted from the transmitter 210 may be referred to as "first light". Part (e.g., reference light 320) of the first light 310 emitted from the transmitter 210 may be received by a light receiving device(s) (e.g., the first light receiving device DET 1) of the receiver 220. As used herein, first receiver 222 (e.g., the first light receiving device DET 1) receiving the first light 310 may be referred to as a reference light receiving device, first light receiving device, or in other various terms. As used herein, another light receiving device(s) (e.g., the second light receiving device DET 2, the third light receiving device DET 3, and the fourth light receiving device DET 4) other than the first light receiving device (e.g., the first light receiving device DET 1) of the receiver 220 may be referred to as a second receiver 224, other (or remaining) light receiving device(s), or in other various terms for ease of description. As used herein, part of the first light 310 emitted from the transmitter 210, which enters the reference light receiving device, may be referred to as reference light 320. The second receiver 224 (e.g., the second light receiving device DET 2, the third light receiving device DET 3, and the fourth light receiving device DET 4) may receive light (which may be referred to herein as second light 330) that is emitted from the transmitter 210 (e.g., a light emitting device), strikes a user 300 (or undergoes a tissue-light interaction), and then is reflected back to the sensor module 200 by at least part of the body of the user 300. As shown in FIG. 3A, "receiver" (e.g., a light receiving module) is briefly shown as a DET. The first wall 240 may be made or formed of rubber. However, this is merely an example, and the housing 230 may be formed of other various materials such as polyethylene, polyester, melamine resin, phenolic resins, polyurethane, glass fiber, asbestos, or porcelain. The housing 230 may be surface-coated with a light reflecting material. The first receiver 222 (e.g., the first light receiving device DET 1) may include one or more light receiving devices or one or more ultrasound wave receiving devices. The second receiver 224 (e.g., the second light receiving device DET 2, the third light receiving device DET 3, and the fourth light receiving device DET 4) may include one or more light receiving devices or one or more ultrasound wave receiving devices.

According to an embodiment, an opposite device may be provided between the transmitter 210 and the first receiver 222 to guide the first light 310 to the first receiver 222 so that the reference light 320 may be received by the first receiver 222. The optical guide may connect the transmitter 210 and the first receiver 222 through a space between the first wall 240 and the light path changer 206. The optical guide may be made or formed of, e.g., optical fiber. When there is an optical guide, the light path changer 206 may be omitted. The first receiver 222 and the second receiver 224 may operate independently (e.g., at different times), simultaneously, or sequentially.

Referring to FIG. 3B, the sensor module 200 may further include an optical structure 342a to cover at least a top portion of the housing 230. FIG. 3B illustrates an example in which the optical structure 342a fully covers the opening of the housing 230. The optical structure 342a may include a total internal reflection (TIR) Fresnel lens. The first light 310 may be redirected through the optical structure 342a. For example, according to an embodiment, the first light 310 may be redirected through the optical structure 342a to the user 300. The second light 330 may be redirected through the optical structure 342a to the receiver 220 to, e.g., be received by the receiver 220. The optical structure 342a may include other various optical structures (e.g., lenses) capable of redirecting the first light 310 and/or second light 330 other than the TIR Fresnel lens. A cover glass 344 may be disposed on the top of the optical structure 342a so as to be in contact with the optical structure 342a. Although FIG. 3B illustrates that the cover glass 344 is disposed in contact with the optical structure 342a, this is merely an example. The cover glass 344 may also cover the top of the optical structure 342a with a preset gap (e.g., an air gap) interposed between the cover glass 344 and the optical structure 342a (and the top of the housing 230). The optical structure 342a may be included in a top portion of at least one of the first area 202 or the second area 204.

Referring to FIG. 3C, in an embodiment not falling under the scope of the claims, the first receiver 222 may be disposed adjacent to the transmitter 210 in the first area 202. The first light 310 emitted from the transmitter 210 may be directed to the outside (e.g., towards the user 300). The reference light 320 emitted from the transmitter 210 may be received by the first receiver 222 disposed in the first area 202. FIG. 3C illustrates an example in which the reference light 320 is reflected by the internal surface of the housing 230 and is received by the first receiver 222. The reference light 320 may be emitted from the transmitter 210 and received by the first receiver 222 directly (or without being reflected by the internal surface). Other light receiving devices (e.g., the second light receiving device DET 2, the third light receiving device DET 3, and the fourth light receiving device DET 4) of the receiver 220 may be disposed in the second area 204. The receiver 220 disposed in the second area 204 may receive the second light 330 reflected by the user 300.

Referring to FIG. 3D, the first receiver 222 and the second receiver 224 may be spaced apart from each other at a preset gap in the second area 204. The wall extending from the light path changer 206 may be disposed in the gap. As the first receiver 222 and the second receiver 224 are spaced apart at a preset gap and the wall is disposed in the gap as shown in FIG. 3D, the reference light emitted from the transmitter 210 may be prevented from being received by at least one of the other light receiving devices.

Referring to FIG. 3E, a second wall 350 may be provided between the first receiver 222 and the second receiver 224. In a case of the optical sensor module 200 as shown in FIG. 3E, the light path changer 206 may be omitted. The second wall 350 may extend up to the top of the housing 230 as shown in FIG. 3E, and, thus, connects to the housing 230. The structure with the second wall 350 may prevent the reference light 320 from being received by the second receiver 224 (e.g., the second light receiving device DET 2, the third light receiving device DET 3, and/or the fourth light receiving device DET 4). The structure with the second wall 350 may prevent the second light 330 from being received by the first receiver 222 (e.g., the first light receiving device DET 1).

The bold solid lines shown in FIGs. 3A to 3E that indicate, e.g., the reference light 320 and the first light receiving device DET 1 are merely used to emphasize the reference light 320 being received by the first light receiving device DET 1, but do not indicate that the amount (or intensity) of the reference light 320 is greater than the light directed to the user 300. Light emitted to the outside (e.g., towards the user 300) may be the same or a different quantity (or intensity) as the reference light 320. This may likewise apply when ultrasound waves are adopted.

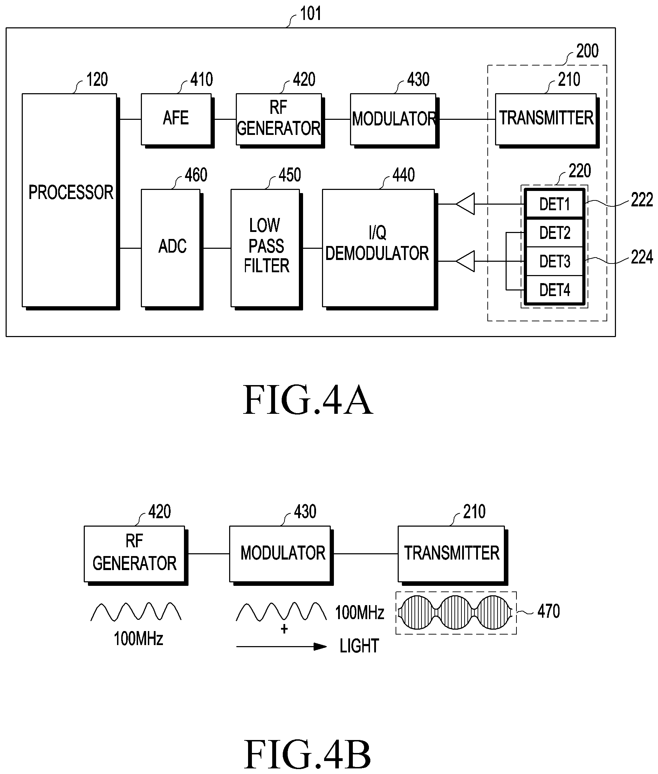

FIGs. 4A and 4B are block diagrams illustrating light modulated as per frequency, which is output from an electronic device 101, according to an embodiment.

Referring to FIG. 4A, the electronic device 101 may include a processor 120, a sensor module 200, an analog front end (AFE) 410, a radio frequency (RF) generator 420, a modulator 430, an in-phase and quadrature (I/Q) demodulator 440, a low pass filter 450, and an analog-digital converter (ADC) 460.

The processor 120 may drive, e.g., software (e.g., a program 140) to control at least one other component (e.g., a hardware or software component) of the electronic device 101 connected with the processor 120 and may process or compute various data.

The sensor module 200 (e.g., an optical sensor module) may include a transmitter 210 (e.g., a light emitting device including a VCSEL) and a receiver 220 (e.g., a first light receiving device DET 1 222, a second light receiving device DET 2, a third light receiving device DET 3 224, and a fourth light receiving device DET 4). The light receiving devices shown in FIG. 4A are an example to describe various embodiments of the present disclosure, but the present disclosure is not intended to be limited to what is shown in FIG. 4A.

The AFE 410 may control the operation of the sensor module 200. The AFE 410 may perform pre-processing to compute and process analog signals in the digital domain. The AFE 410 may include an amplifier to amplify analog signals entered. The AFE 410 may convert the entered analog signals into digital signals and output the digital signals.

The RF generator 420 may generate frequencies (or frequency signals) in a MHz-GHz range. Various functions/operations performed by the RF generator 420 may be controlled by the processor 120.

The modulator 430 may include a bias-tee. The modulator 430 (e.g., a bias-tee) may synthesize a voltage (e.g., a direct-current (DC) voltage) provided from a voltage supplying module and a frequency signal (e.g., an RF signal) generated by the RF generator 420. The modulator 430 may transmit the synthesized (or modulated) signal to the transmitter 210. The transmitter 210 may output light that has been modulated to have a designated frequency based on the synthesized signal. The modulator 430 may include a first input terminal to receive a voltage, a second input terminal to receive frequency signals, and an output terminal to transmit synthesized signals to the transmitter 210. However, the voltage provided from the voltage supplying module may be provided to the transmitter 210 directly (or without being transmitted to the modulator 430).

The modulator 430 may include various circuits (or modules) to synthesize frequency signals (e.g., RF signals) and voltage under the control of the processor alternatively or in exchange for the bias-tee. In this case, at least some functions performed by the RF generator 420 may be performed by the modulator 430, or at least some functions performed by the modulator 430 may be performed by the RF generator 420.

The modulator 430 may be omitted. In this case, the frequency signal generated by the RF generator 420 under the control of the processor may be directly transmitted to the transmitter 210. The processor 120 may control the transmitter 210 so that the light modulated as per the frequency signal generated by the RF generator 420 may be output through the transmitter 210.

The I/Q demodulator 440 may split the waveform of the second light 330 received by the receiver 220 into real information and imaginary information. Various functions/operations performed by the I/Q demodulator 440 may be controlled by the processor 120.

The low pass filter 450 may receive signals separated by the I/Q demodulator 440 and pass the signal of the frequency band lower than a designated frequency among the received signals.

The ADC 460 may convert analog signals into digital signals. Various functions/operations performed by the ADC 460 may be controlled by the processor 120.

FIG. 4B is a block diagram in which modulated light is output through the transmitter 210.

Referring to FIG. 4B, the processor 120 may control the RF generator 420 to output a designated frequency (e.g., 100MHz). The designated frequency may be determined by the processor 120. The designated frequency may be determined by a modem (or a communication processor) in order to be generated (or output) by the RF generator 420 or a transceiver transmit end (TX). The RF generator 420 and the transceiver TX may be connected together via a coupler so that the designated frequency is generated (or output) by the transceiver TX. The modulated light 470 may indicate light that periodically varies in intensity depending on the designated frequency and is output, as shown in FIG. 4B. The light output while varying periodically may be in the form of a sinewave (or sinusoidal wave). A modulation process/operation may change light emitted in a straight line into a sinusoidal waveform.

The RF generator 420 and the I/Q demodulator 440 may separately (or additionally) be provided to various functions/operations of the sensor module 200. The RF generator 420 and the I/Q demodulator 440 of the electronic device 101 may functionally be shared to perform the functions/operations of the sensor module 200 so as to perform other functions/operations of the electronic device 101. To that end, a port not used for communication among the plurality of ports of the RF generator 420 may be used to perform various functions/operations of the sensor module 200 (e.g., an optical sensor module). For example, at least some component of the electronic device 101 may be connected to the RF generator 420 through a port not used for communication.

At least some components of the electronic device 101, as shown in FIGs. 4A and 4B, may be integrated with, or included in, the processor 120. At least some components (e.g., the AFE 410, the RF generator 420, the modulator 430, the sensor module 200, the I/Q demodulator 440, the low pass filter 450, and the ADC 460) of the electronic device 101 may be manufactured in a single sensor package.

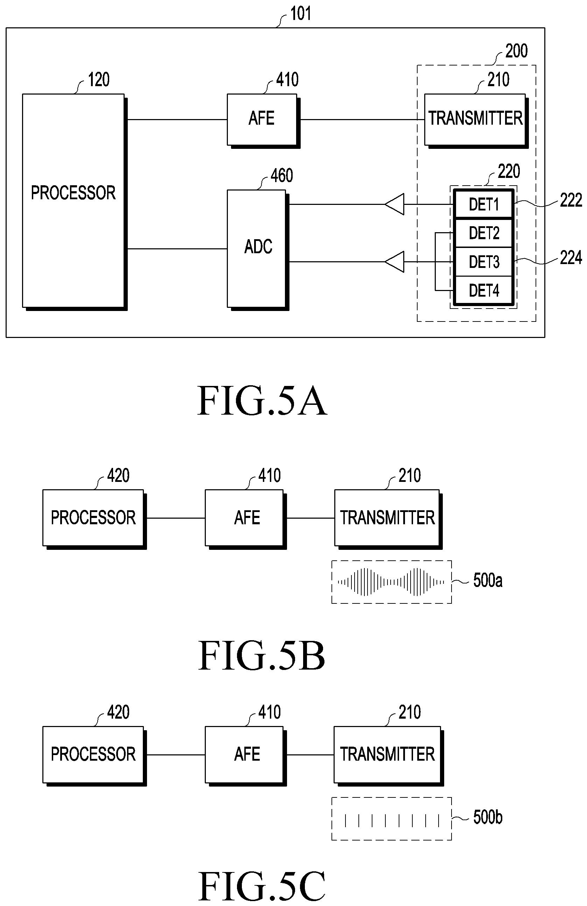

FIGs. 5A and 5B are block diagrams illustrating pulses of light that are modified in height and/or width and which are output from an electronic device 101 according to an embodiment. FIG. 5C is a block diagram illustrating pulses of light with a same amplitude or strength and which are output from an electronic device 101 according to an embodiment.

Referring to FIG. 5A, an electronic device 101 may exclude the RF generator 420, modulator 430, I/Q demodulator 440, and low pass filter 450 from the electronic device 101 of FIG. 4A.

As the RF generator 420 and the modulator 430 are omitted, the processor 120 may change the height and width of light pulses emitted from the transmitter 210 to allow the first light 310 to be output in a particular waveform and may control the transmitter 210 to sequentially output the light pulses 500a that are changed in height and width, as shown in FIG. 5B. The processor 120 may control the transmitter 210 to output light pulses 500b that are the same in height and width as shown in FIG. 5C. As used herein, the phrase "light beams are sequentially output" may be interchangeably used with the phrase "light beams are output in a group."

Upon receiving second light 330 corresponding to the first light 310 output in a particular waveform (e.g., a sinusoidal or square waveform), the processor 120 may compare the first light 310 and the second light 330 and obtain biometric information based on the results of comparison. The processor 120 may transmit an instruction to the AFE 410 in order to change the height and/or width of light emitted from the transmitter 210 so as to output the first light 310 in a particular waveform.

The I/Q demodulator 440 may split the waveform of measurement into two (e.g., the above-described real information and imaginary information) in order to determine a change in the amplitude and phase of the second light 330 (or the first light 310) which continuously varies. The low pass filter 440 may pass signals of a lower frequency band among at least some of the split signals.

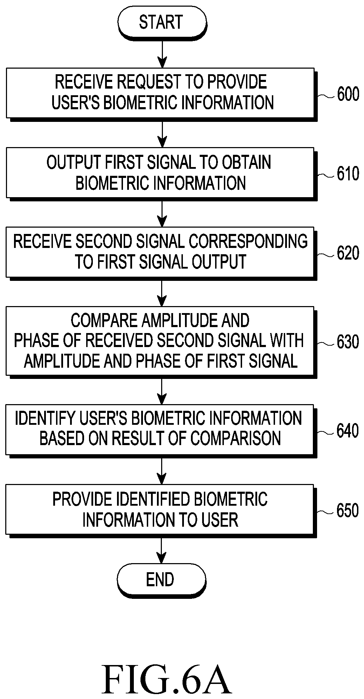

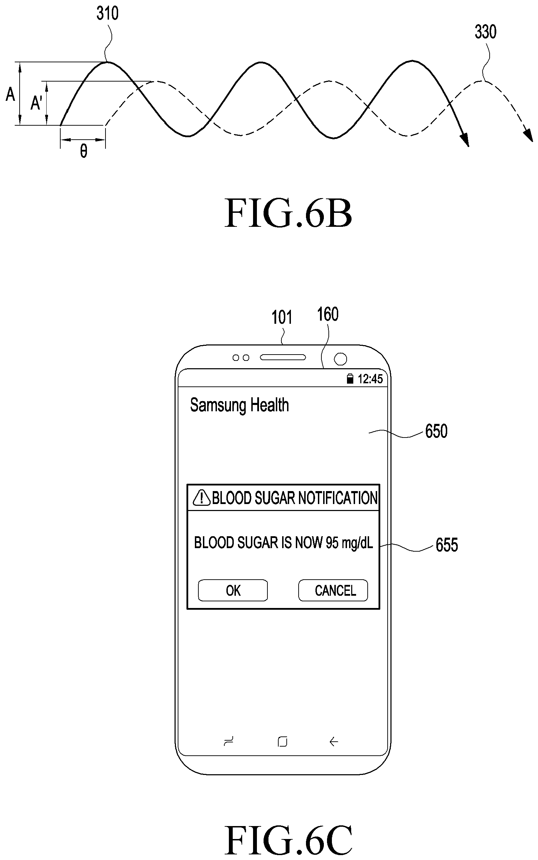

FIGs. 6A, 6B, and 6C are a flowchart, a graph, and an illustration, respectively, illustrating an electronic device's 101 function/operation for providing biometric information based on variations in amplitude and phase according to an embodiment.

Referring to FIG. 6A, in a method for operating the electronic device 101, an electronic device (e.g., the processor 120 of FIG. 1) may receive a request to provide biometric information from a user using an input device (e.g., the display device 160 of FIG. 1) in step 600. The request to provide biometric information may include the user's touch input (including hovering information) and/or the user's voice input. The request to provide biometric information may include periodic or aperiodic inputs.

The electronic device (e.g., the processor 120) may output a first signal (e.g., first light 310) to obtain biometric information using a transmitter (e.g., a light emitting device, the transmitter 210) in step 610. The operation of outputting the first signal (e.g., the first light 310) may include various operations for outputting modulated light through the transmitter 210.

The processor 120 may select a frequency ranging from 1MHz to 1GHz as the frequency generated (or output) by the RF generator 420, but the present disclosure is not intended to be limited thereto. When the transmitter 210 includes a plurality of light emitting devices, each of which emits one of different wavelengths of light, the processor 120 may select at least one of the wavelengths of light having a wavelength band corresponding to a target biometric information (e.g., fat and water) that the processor 120 intends to obtain, from among the plurality of light emitting devices. The target biometric information may be received from the user. The wavelength band corresponding to the target biometric information may be previously designated. The wavelength band corresponding to the target biometric information may be selected by the user.

The electronic device (e.g., the processor 120) may receive a second signal (e.g., the second light 320) corresponding to the first signal (e.g., the first light 310) output from the transmitter 210 by using the receiver 220 in step 620.

The electronic device (e.g., the processor 120), in step 630, may compare the amplitude and phase of the first signal with the second signal which is emitted to the user 300, undergoes a tissue-light interaction, and is then reflected back to the electronic device (e.g., the receiver 220). The electronic device 101 may compare first data for the first light 310 emitted from the transmitter 210 with second data for the second light 330 reflected by the user 300 and received by the electronic device (e.g., the receiver 220). The electronic device (e.g., the processor 120) may determine that the amplitude of the second light 330 has been changed from A to A' and the phase is shifted by $\theta$. FIG. 6A illustrates an example of comparing the first light 310 and the second light 330 in light of both amplitude and phase. However, the electronic device 101 may include the step of comparing the first light 310 and the second light 330 in light of either phase or amplitude. The electronic device (e.g., the processor 120) may obtain biometric information based on the result of the comparison of any one of phase and amplitude. The electronic device (e.g., the processor 120) may obtain biometric information based on the result of the comparison of both amplitude and phase.

When the light emitted from the sensor module 200 has a constant height and width, an electronic device (e.g., the processor 120), in step 630, may use a histogram for an arrival time of the second light 330 which undergoes a tissue-light interaction, the light is then reflected by the user 300, and is received by the electronic device (e.g., the receiver 220) to calculate an absorption coefficient and scattering coefficient of the user's body tissue or to measure variations in biometric information in the user's body tissue. Various techniques may apply to obtain the user's biometric information using the information about the arrival time of the second light 330 or the histogram for the arrival time. As an example, information associated with an in-vivo scattering coefficient may be obtained using a magnitude of a peak and a shift in peak, and information associated with the in-vivo absorption coefficient may be obtained from a slope connecting bars on a right side of the peak in the histogram.

The electronic device (e.g., the processor 120) may identify the user's biometric information based on the result of the comparison in step 640. Various techniques to obtain biometric information (or determine a variation in biometric information) based on the amplitude and/or phase of the received (or obtained) light (e.g., the first light 310 and/or second light 330) may apply to step 640. For example, a memory of the electronic device 101 may previously store a mapping table including data regarding phase transitions and/or amplitude attenuation of the first light 310 emitted from the transmitter 210 and data regarding biometric information corresponding to the phase transitions and/or amplitude attenuation. The processor 120 may obtain the user's biometric information based on the mapping table.

The electronic device (e.g., the processor 120) may provide the obtained biometric information to the user using the display device (e.g., the display device 160), in step 650. The electronic device 101 may display a biometric information notification message 655 containing the obtained biometric information (e.g., glucose level or blood sugar level) through an execution screen 650 of a designated application (e.g., Samsung® Health™ application) as shown in FIG. 6C. The electronic device 101 may only display the biometric information notification message 655 without using the designated application (e.g., by having a message pop up on the home screen).

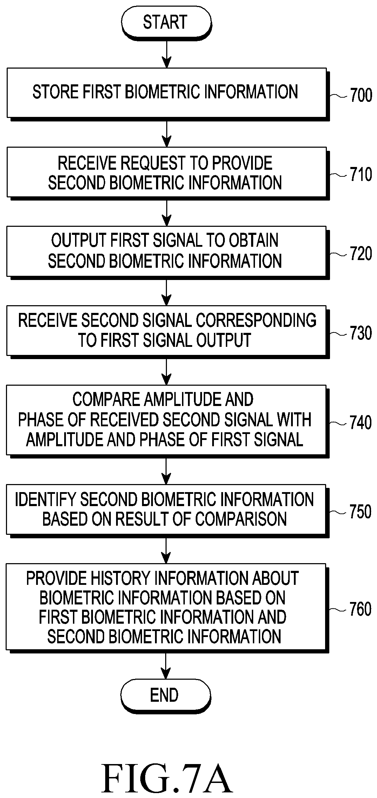

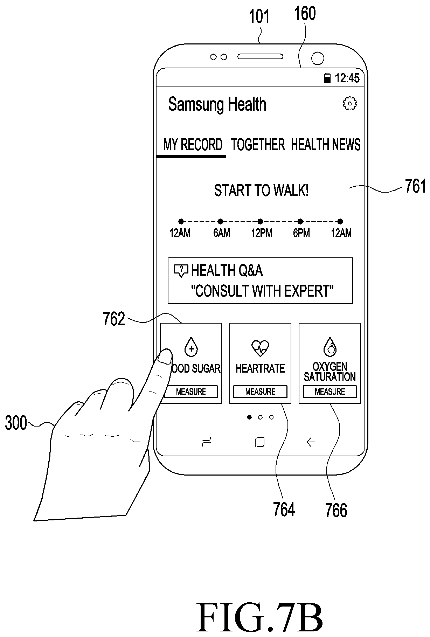

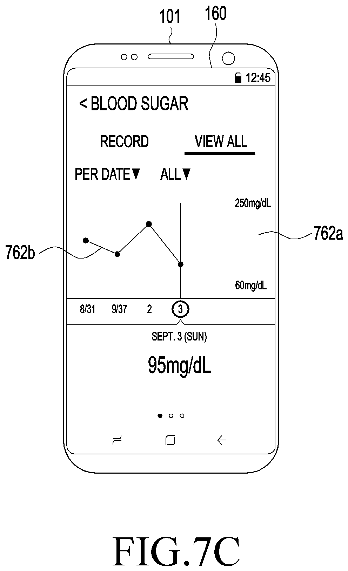

FIGs. 7A, 7B, and 7C are a flowchart and illustrations of an electronic device's 101 function/operation for providing comparison data regarding biometric information according to an embodiment.

Referring to FIG. 7A, an electronic device (e.g., the processor 120) may store first biometric information using a storage device (e.g., the memory 130) in step 700. In step 700, the stored first biometric information may include biometric information that was obtained at a particular time (e.g., yesterday) in the past and stored in the electronic device 101.

The electronic device (e.g., the processor 120) may receive a request to provide second biometric information using an input device (e.g., the display device 160) in step 710. The input to obtain the second biometric information may include the user's touch and/or voice input. The input to obtain the second biometric information may include periodic or aperiodic inputs.

The electronic device (e.g., the processor 120) may output a first signal to obtain the second biometric information using a transmitter 210 in step 720. The step of outputting the first light 310 may include the step of determining at least one frequency (e.g., 100MHz) for the electronic device (e.g., the processor 120) to output the first light 310. The electronic device 101 may determine at least one frequency based on the user's input or an instruction to select at least one frequency as previously programmed. The processor 120 may control the RF generator 420 to generate a frequency ranging from, e.g., 1MHz to, e.g., 1GHz. However, the range from 1MHz to 1GHz is merely an example to describe various embodiments of the present disclosure, and the processor 120 may alternatively control the RF generator 420 to generate a frequency exceeding 1GHz. The processor 120 may control the transmitter 210 to generate light pulses constant in height or width.

When the transmitter 210 includes a plurality of light emitting devices, each of which emits one of different wavelengths of light, the processor 120 may select at least one of the emitted wavelengths of light from among the plurality of light emitting devices based on target biometric information (e.g., body water percentage) that the processor 120 intends to acquire.

The electronic device (e.g., the processor 120) may receive a second signal corresponding to the first signal output from the transmitter 210 using the receiver 220 in step 730.

The electronic device (e.g., the processor 120), in step 740, may compare the amplitude and phase of the first signal with the second signal which, after a tissue-light interaction, is reflected by the user 300 and is then received by the electronic device (e.g., the receiver 220). The electronic device 101 may compare the first light 310 emitted from the transmitter 210 with the second light 330 reflected by the user 300 of Fig. 3a) after the tissue-light interaction and received by the electronic device (e.g., the receiver 220), and determines that the second light 330 has been shifted from A to A' and the phase has been shifted by θ as shown in FIG. 6B. Although FIG. 6A illustrates an example in which the electronic device 101 makes a comparison of both amplitude and phase, the electronic device 101 may alternatively compare only on either phase or amplitude. The electronic device (e.g., the processor 120), in step 740, may measure the absorption coefficient and/or scattering coefficient for the user's body tissue using information about the arrival time (or time lapse from the output to re-reception) of the second signal which is, after a tissue-light interaction, reflected by the user 300 and is then reflected back to the electronic device (e.g., the receiver 220). There may be applied various methods for measuring the absorption coefficient and/or scattering coefficient for the user's body tissue using information (e.g., a histogram) about the arrival time of a signal (e.g., the second light 330), e.g., a method of using a mapping table defining the relationship between the arrival time of the output signal and the absorption coefficient and/or scattering coefficient.

The electronic device (e.g., the processor 120), in step 750, may identify the user's second biometric information based on the result of the comparison obtained in step 740. Step 750 may adopt various techniques to obtain the second biometric information (or determine variations in biometric information) using the data about variations in amplitude and/or phase of light (e.g., the first light 310 and/or second light 330) which is, after a tissue-light interaction, reflected by the user 300 and is then received by the electronic device (e.g., the receiver 220). For example, a memory of the electronic device 101 may previously store a mapping table including data regarding phase transitions and/or amplitude attenuation of the first light and second light 330 output and data regarding biometric information corresponding to the phase transitions and/or amplitude attenuation. The processor 120 may obtain the user's biometric information based on the mapping table.

The electronic device (e.g., the processor 120), in step 760, may provide history information about the user's biometric information based on the first biometric information and second biometric information and by using the display device 160. The electronic device 101 may display an execution screen 761 of an application related to providing the user's biometric information. The execution screen 761 of the application may be displayed on the electronic device 101 as per a designated application execution request. Alternatively, when the second biometric information is obtained, the processor 120 may display the application execution screen 761 on the electronic device 101 regardless of whether the application execution request is received (or automatically), or may, without displaying, store the same in the memory of the electronic device 101. The application execution screen 761 may display various interfaces to measure (or obtain or identify) various pieces of biometric information, e.g., at least one of an interface 762 to measure glucose level, an interface 764 to measure heart rate, and an interface 764 to measure oxygen saturation. Other various interfaces for identifying biometric information other than the interfaces 762, 764, and 766 shown in FIG. 7B may be interchangeably/additionally displayed on the electronic device 101. The electronic device 101 may receive an input to identify information about glucose level from the user 300 as shown in FIG. 7B. The input to identify the information about glucose level may include a touch input to the interface 762. The electronic device 101 may display glucose history information 762b on a screen 762a to provide glucose information according to the input to the interface 762, as shown in FIG. 7C.

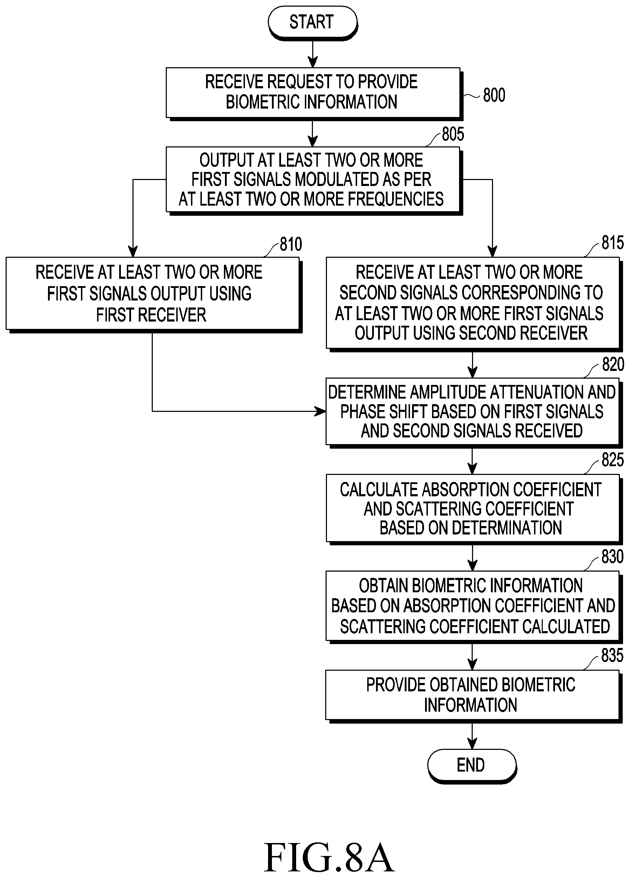

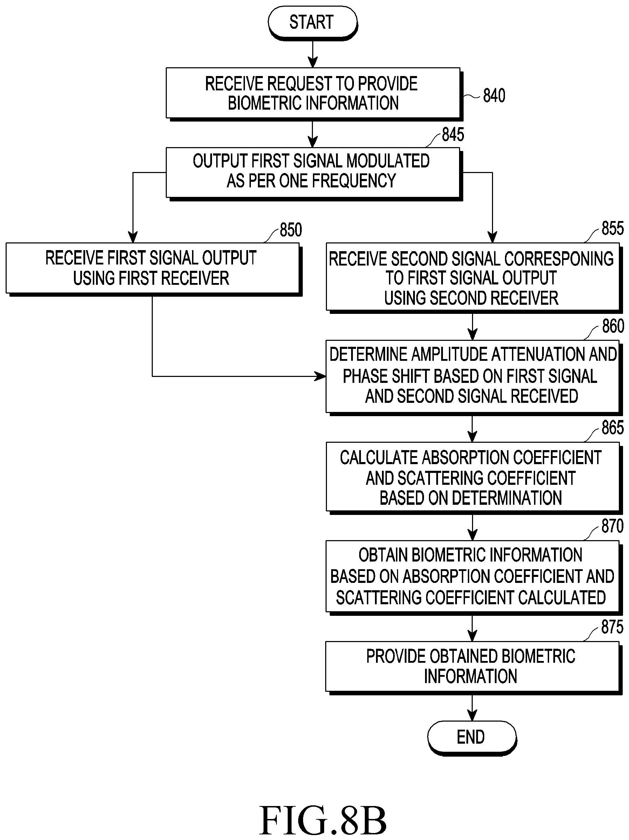

FIGs. 8A and 8B are flowcharts of an electronic device's 101 function/operation for obtaining biometric information using an absorption coefficient and a scattering coefficient and providing the obtained biometric information, according to an embodiment.

Referring to FIGs. 8A and 8B, an electronic device (e.g., the processor 120) may receive a request to provide biometric information using an input device (e.g., the display device 160) in step 800. Step 800 may be a triggering condition to perform step 805. The trigger condition may further include at least one of when a designated time arrives, when a preset measurement interval (e.g., a one-hour gap) arrives, when a designated circumstance is detected (e.g., where the user finishes her meal or exercise), and when a command to perform measurement is received from an external electronic device.

The electronic device (e.g., the processor 120), in step 805, may output at least two or more first signals that have been modulated as per at least two or more frequencies.

The electronic device (e.g., the processor 120), in step 805, may sequentially output at least one or more first light beams that have been modulated as per at least two or more frequencies, using the transmitter 210. The first light beams output in step 805 may include light beams with the same wavelength (e.g., 940nm) but different modulated frequencies (e.g., 50MHz and 100MHz). When the transmitter 210 includes a plurality of light sources to output light beams with the same wavelength, the electronic device 101 may control the plurality of light sources to simultaneously output light beams modulated by at least two or more frequencies.

The electronic device (e.g., the processor 120), in step 810, may receive at least two or more first signal outputs (e.g., the first light 310) using the first light receiving device 222.

The electronic device (e.g., the processor 120), in step 815, may receive at least two or more second signals (e.g., the second light 330) corresponding to at least two or more first signal outputs (e.g., the first light 310) using the second receiver (e.g., the second light receiving device DET 2, the third light receiving device DET 3, and the fourth light receiving device DET 4 of FIG. 2A). Steps 810 and 815 may be performed simultaneously or sequentially.

The electronic device (e.g., the processor 120), in step 820, may determine amplitude attenuation and phase shift based on the first signal and the second signal received by the receiver 220. The electronic device 101 may only determine ether the amplitude attenuation or the phase transition.

The electronic device (e.g., the processor 120), in step 825, may calculate the absorption coefficient and scattering coefficient based on the determination made in step 820. A diffusion equation or diffusion approximation to the radiative transport equation may be used to calculate the absorption coefficient and scattering coefficient. As per the diffusion equation, variation rates of amplitude and variation rates of phase may be calculated based on at least two or more frequencies, and values obtained by applying a non-linear least-squares fitting to the calculated values, which are most similar to the variation rate of amplitude and variation rate of phase theoretically calculated, may be used to calculate the absorption coefficient and scattering coefficient. In this case, boundary conditions (e.g., extrapolated boundary conditions) most fitting the circumstance of measurement may apply. In relation to step 825, various techniques may apply to the method of calculating the absorption coefficient and scattering coefficient. The absorption coefficient may be calculated by the diffusion equation, independently from, or regardless of, the scattering coefficient. In obtaining the user's biometric information, the electronic device 101 may obtain the biometric information based on the absorption coefficient and/or the scattering coefficient.

The electronic device (e.g., the processor 120), in step 830, may obtain the biometric information based on the absorption coefficient and scattering coefficient calculated in step 825. The electronic device (e.g., the processor 120) may also obtain the biometric information based on one or more of the calculated absorption coefficient, the calculated scattering coefficient, the arrival pattern of photons measured in the histogram and the degree of variation in amplitude as identified, and the variation in phase. When it comes to glucose, the processor 120 may identify a current quantity of glucose or variation in the quantity of glucose using the absorption coefficient and/or scattering coefficient or phase shift (or phase difference) and/or the arrival pattern (e.g., arrival time) of photons measured in the histogram. The electronic device (e.g., the processor 120) may identify the quantity of glucose or variations in the quantity of glucose based on the mapping table including the correspondence between the quantity of glucose and the phase or phase shift to measure the quantity of glucose using the scattering coefficient or arrival pattern of photons measured in the histogram, phase, and phase transitions. There may be various methods for measuring the quantity of glucose using at least one of the absorption coefficient, scattering coefficient, phase, and phase shift (or phase difference).

Various techniques may be applied to step 830 to obtain biometric information based on the absorption coefficient and scattering coefficient. For example, the memory of the electronic device 101 may previously store a mapping table including data of biometric information corresponding to the absorption coefficient and/or the scattering coefficient. The processor 120 may obtain the user's biometric information based on the mapping table.

The electronic device (e.g., the processor 120) provides the obtained biometric information to the user using the display device 160, in step 835.

FIG. 8B is a flowchart of a method in which the electronic device 101 uses light modulated as per a single frequency, unlike in the embodiment shown in FIG. 8A, when the processor 120 performs modulation (e.g., as shown in FIG. 4A or FIG. 4B) using a low frequency (e.g., 10MHz) directly or through the RF generator 420. When a low frequency is used, the amplitude and phase of light (e.g., the first light 310 and/or second light 330 of FIG. 3A) respond linearly as the frequency varies. Thus, a diffusion equation expressed for low frequency bands (e.g., frequency domain solutions to diffusion equation) may be put to use.

When a low frequency is used, if the product of the angular speed ($\omega$) used for modulation and the optical absorption relaxation time (which is the reciprocal of the product of the speed of light (c) in the tissue and the speed of light (c), i.e., $\tau = 1/(\mu_a \cdot c)$) is smaller than 0.7, (i.e., $\omega\tau < 0.7$), Equations (1) and (2) below may be used.

$$\phi = \frac{\sqrt{\frac{3}{2}}\mu_s'}{c\sqrt{2\mu_a}} r\omega \qquad \qquad \ldots(1)$$

$$-\ln(m) = \frac{\sqrt{\frac{3}{2}}\mu_s'}{c\sqrt{2\mu_a}} \times \frac{r\omega^2}{4\mu_a c} \qquad \qquad \ldots(2)$$

In Equations (1) and (2) above, $\Phi$ may indicate a phase shift, $m$ may indicate amplitude attenuation, $\mu_a$ may indicate an absorption coefficient, $\mu_s'$ may indicate a scattering coefficient, $r$ may indicate a distance between a light source (e.g., the transmitter 210) and a measuring unit (e.g., the user 300), and ln() may indicate the natural logarithm. Distance $r$ may previously be designated.

The above described equation $\omega\tau < 0.7$ may be a standard of the range in which the modulation frequency is allowable as per the absorption coefficient (or degree of absorption). For example, obtaining the user's biometric information may adopt a lower frequency for lighter skin tones and a higher frequency for darker skin tones. The processor 120 may obtain information about the user's skin tone and may determine the modulation frequency to modulate the light (e.g., the first light 310) output from the electronic device 101 based on the obtained skin tone. In obtaining the user's biometric information, the electronic device 101 may fix (or set) the modulation frequency to a low frequency (e.g., 10MHz) regardless of the status of the measuring target (e.g., the user's skin tone) and may use the fixed low frequency in order to obtain the user's biometric information. In obtaining the user's biometric information, the electronic device 101 may measure the reflectance of light in a steady state (0Hz) where light emitted from the transmitter 210 is reflected by the user's body and received by the receiver 220 and determines the frequency to be used for modulating the light according to the measured reflectance. For example, the electronic device 101 may obtain information regarding the user's melanin pigment based on at least part of the reflectance and the arrival pattern of photons measured in the histogram, phase shift, and amplitude attenuation of light received by the electronic device 101. The electronic device 101 may determine the user's skin tone based on the obtained melanin pigment information. The electronic device 101 may determine the frequency to be used for modulation according to the determined skin tone. Various techniques to measure melanin pigment may apply to the function/operation of obtaining the user's melanin pigment information using at least part of the reflectance, phase shift, and amplitude attenuation of light received by the electronic device 101.

The electronic device (e.g., the processor 120) may measure variations in body composition based on at least one of the scattering coefficient calculated, the arrival pattern of photons measured in the histogram, and the degree of phase shift. For example, the electronic device may identify the current amount of glucose, e.g., in blood, by the obtained phase value, the variation in glucose quantity by the phase shift, and the amount of glucose or variation therein by the scattering coefficient or the arrival pattern of photons measured in the histogram. Variations in body composition corresponding to an extinction coefficient of body composition may be measured based on the extinction coefficient. The electronic device 101 may obtain biometric information regarding body composition/components of body composition (or body components), each of which corresponds to one of wavelengths used, depending on the number of the wavelengths used. The electronic device 101 may determine variations in the total amount of biometric information regarding body composition/body components. Per Beer-Lambert's Law, the extinction coefficient $\mu_a(\lambda)$ for the wavelength $\lambda$ of light used may be calculated in Equation (3) below, based on a linear combination of a product of percent concentration or molar concentration of a target body component (e.g., water or fat) and information known for target body components (e.g., fat, water, blood, or glucose), i.e., extinction coefficient. In Equation (3) below, the body composition/body components other than water or fat are referred to as "Others." In other words, for other body composition/body components, as well as water or fat, the linear combination of the product of percent concentration or molar concentration of the target body component and the extinction coefficient may apply to Equation (3) below:

$$\mu_a(\lambda) = \text{extinction coefficient}(\lambda)_{\text{water}} \times \text{concentration}_{\text{water}} + \text{extinction}$$

$$\text{coefficient}(\lambda)_{\text{fat}} \times \text{concentration}_{\text{fat}} + \text{Others} \qquad \ldots(3)$$

In determining the percent or molar concentration of body composition/body components, various techniques may apply to calculate the percent or molar concentration of the target body component using information about variations in, e.g., amplitude and/or phase, of light (e.g., the second light 330) reflected by the user's body and received. For example, the percent or molar concentration of the target body component may be measured based on the mapping table including data regarding the percent or molar concentration corresponding to the amplitude attenuation or phase shift, which is stored in the memory 130. The electronic device (e.g., the processor 120) may measure variations in body composition based on at least one of the scattering coefficient calculated, and the degree of phase shift. For example, the electronic device may identify the current amount of glucose, e.g., in blood, by the obtained phase value, the variation in glucose quantity by the phase shift, and the amount of glucose or variation therein by the scattering coefficient. When the transmitter 210 s configured to be a white LED or a tunable light source, the electronic device 101 may further include a spectrometer to calculate the absorption coefficient and the scattering coefficient of light corresponding to each of the plurality of body components. When the transmitter 210 includes a plurality of light sources, each of which is configured to only output a single particular wavelength, the electronic device may individually drive the light sources, each of which corresponds to one of the plurality of pieces of target biometric information, and simultaneously or sequentially output light beams to the user 300 in order to calculate the absorption coefficient and scattering coefficient of light corresponding to each of the plurality of body components.

Referring to FIG. 8B, according to an embodiment, in a method for operating an electronic device 101, the electronic device (e.g., the processor 120) may receive a request to provide biometric information using an input device (e.g., the display device 160) in step 840.

The electronic device (e.g., the processor 120) may output a first signal, which has been modulated as per one frequency, using a transmitter 210 in step 845.

The electronic device, in step 850, may receive the first signal from the transmitter 210, using a first receiver (e.g., the first light receiving device DET 1).

The electronic device (e.g., the processor 120), in step 855, may receive a second signal corresponding to the first signal from the transmitter 210, using a second receiver (e.g., the second light receiving device DET 2, the third light receiving device DET 3, and/or the fourth light receiving device DET 4 of Fig. 3a or additional light receiving device(s)).

According to an embodiment, in the method for operating the electronic device 101, the electronic device (e.g., the processor 120), in step 860, may determine amplitude attenuation and phase shift based on the received first signal and the received second signal.

The electronic device (e.g., the processor 120), in step 865, may calculate the absorption coefficient and scattering coefficient based on the determination made in step 860.

The electronic device (e.g., the processor 120), in step 870, may obtain the biometric information based on the absorption coefficient and scattering coefficient calculated. The electronic device 101 may obtain the biometric information based on the calculated absorption coefficient or the calculated scattering coefficient. The histogram-related description, which has been made in connection with step 830, may apply likewise to step 870.

The electronic device may provide the biometric information, which is obtained in step 870, to the user using the display device 160, in step 875.

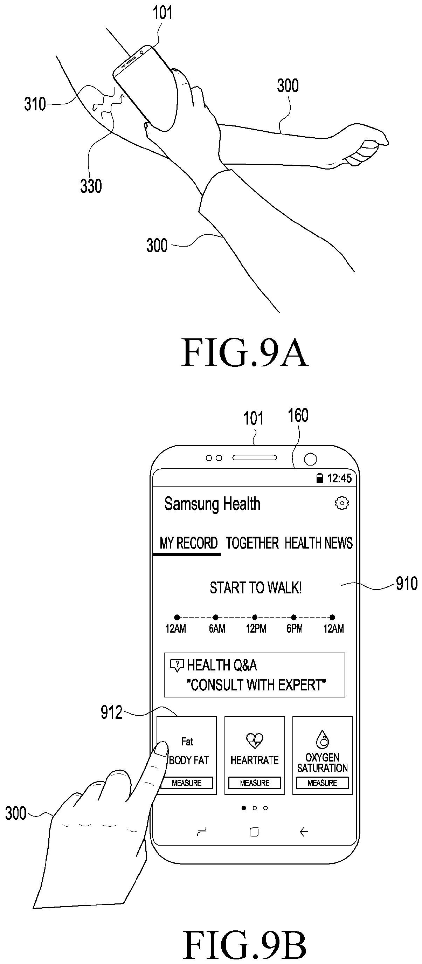

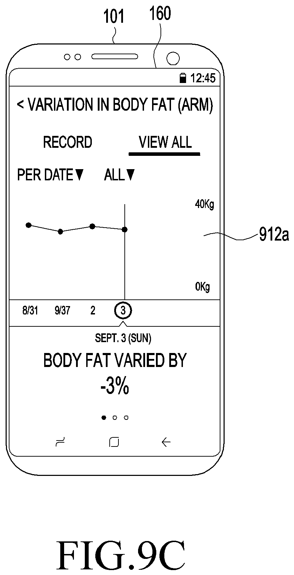

FIGs. 9A, 9B, and 9C are illustrations of an electronic device's 101 function/operation for obtaining a body fat amount and providing information about the obtained body fat amount, according to an embodiment.

Referring to FIG. 9A, according to an embodiment, the electronic device 101 may detect an approach (or contact) of a part of a user's 300 body to the electronic device 101. Upon receiving an input from the user 300 to request providing biometric information, the electronic device 101 may output the first light 310 to obtain the user's 300 biometric information as shown in FIG. 9A. The electronic device 101 may receive the second light 330 corresponding to the first light 310. The electronic device 101 may provide information regarding body fat distribution at the point where the first light 310 is reflected by the part of the user's 300 body.

Referring to FIG. 9B, the electronic device 101 may receive an input to identify the obtained biometric information (e.g., body fat distribution). The input to identify biometric information may include, e.g., a touch input to a particular interface 912 on the screen 910 to provide biometric information.

Referring to FIG. 9C, the electronic device 101 may display a screen 912a to provide the user 300 with the body fat distribution measured. The screen 912a may display information about the trend of body fat distribution varying on the the part of the user's 300 body around, e.g., left biceps, according to the input to identify biometric information. For example, the electronic device 101 may display, on the screen 912a, history information about the body fat distribution of the part of the user's 300 body.

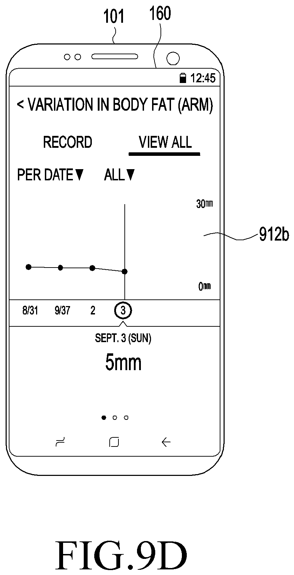

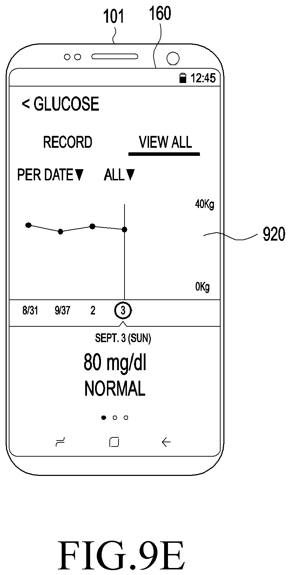

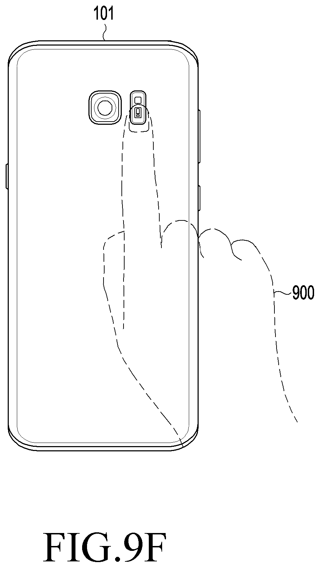

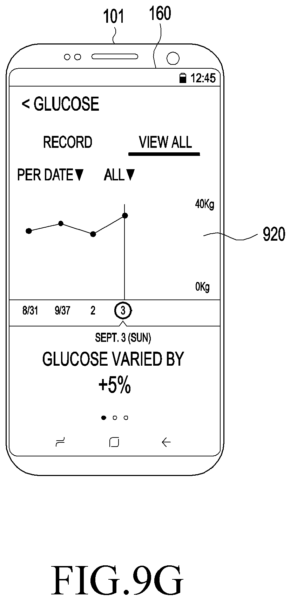

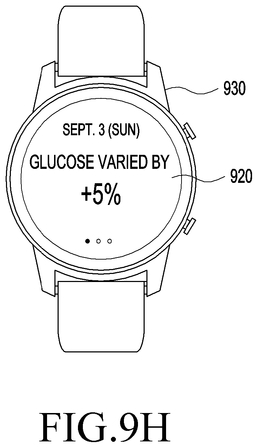

According to an embodiment, light of a wavelength of 930nm may be used to measure body fat distribution. FIGs. 9D, 9E, 9F, 9G, and 9H are illustrations of an electronic device's function/operation for obtaining information about the glucose quantity, or its variation, in blood, and providing information about the obtained glucose quantity or variation, according to an embodiment.

Referring to FIG. 9D, the electronic device 101 may detect an approach (or contact) of a part of a user's 300 body to the electronic device 101. Upon detecting the user's 300 approach, the electronic device 101 may emit (or radiate) a signal (e.g., light) to the user 300 to obtain information about the amount of glucose in blood.

Referring to FIGs. 9E, 9F, and 9H, the electronic device 101 may output, on, e.g., the display or an application execution screen 920, information about the amount of glucose (e.g., 100mg/100ml) or a variation (e.g., 0%) in the amount of glucose obtained by a method according to an embodiment. The electronic device 101 may control an external electronic device (e.g., the wearable device 930) operatively connected with the electronic device 101 as shown in FIGs. 9G and 9H to provide the information about the amount of glucose or variation in the amount of glucose to the user 900 through, e.g., the application execution screen 920. The step of providing the user 900 with the information about the amount of glucose or variation in the amount of glucose may alternatively be controlled by a processor included in the wearable device.

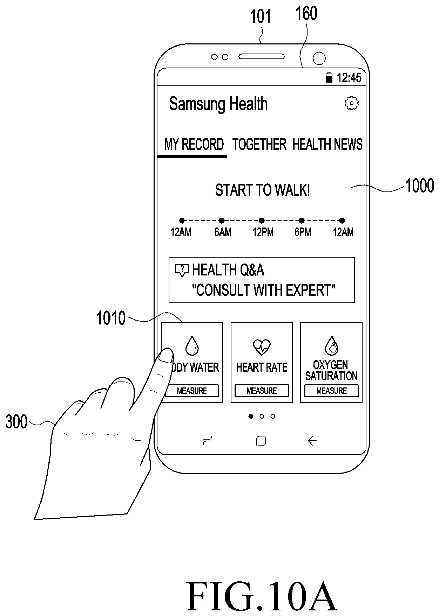

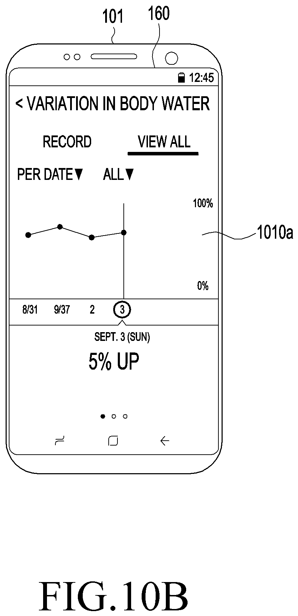

FIGs. 10A and 10B are illustrations of electronic device's 101 function/operation for obtaining an amount of water and providing information about the obtained water quantity, according to an embodiment.

Referring to FIG. 10A, the electronic device 101 may receive an input to provide the amount of water of the user 300. The input to provide the amount of water may include, e.g., a touch input to a particular interface 1010 on the screen 1000 to provide the user's 300 biometric information. The amount of water may indicate the amount of water of a particular part of the body of the user 300 (e.g., around the left biceps) as shown in FIG. 9A. The amount of water may include the amount of water in the user's 300 part of the body (e.g., the left arm) evaluated based on the amount of water measured on the user's 300 particular part of the body (e.g., the part of the body contacted or approached by the sensor module). The amount of water may include the amount of water in the user's 300 whole body estimated based on the amount of water measured on the user's 300 particular part of the body. In this case, various techniques may be applied (e.g., estimation algorithms) to estimate the amount of water (or body water) in the user's 300 entire body or part of the body. The amount of water may be measured in such a manner as shown in FIG. 9A, in which the first light 310 is output and the second light 330 is received.

Referring to FIG. 10B, as per the input to provide the amount of water, the electronic device 101 may display a screen 1010a to provide the user 300 with variations (e.g., 5%) in the amount of water measured according to an embodiment. The variations in the amount of water may include information resulting from the comparison between the amount of water obtained upon the user's 300 input (or at the current time) and the amount of water measured at the latest time (e.g., 24 hours ago). The screen 1010a may display information about the variation in the amount of water (or a relative amount of water) in at least one of the part of the body or the whole body when the amount of water has been measured. The electronic device 101 may display, on the screen 1010a, history information (e.g., the graph shown in FIG. 10B) about the amount of water in the part of the user's 300 body. Although FIG. 10B illustrates an example in which the relative amount of water (or variation in the amount of water) is provided, information regarding the absolute amount of water (e.g., the amount of water (e.g., 50%) measured on the particular part of the body) may alternatively be provided. For example, light of a wavelength of 975nm may be used to measure the amount of water.

The electronic device 101 may measure the amount of water inside the body as well as the amount of water on the skin (e.g., the amount of water in a particular part of the body). Thus, it is possible to provide such information as, e.g., the need for rehydration, e.g., by drinking coffee, the degree of aging, recommending cosmetics based on body water, monitoring health conditions by measuring puffiness due to insufficient body water, or a diet guide related to body water.

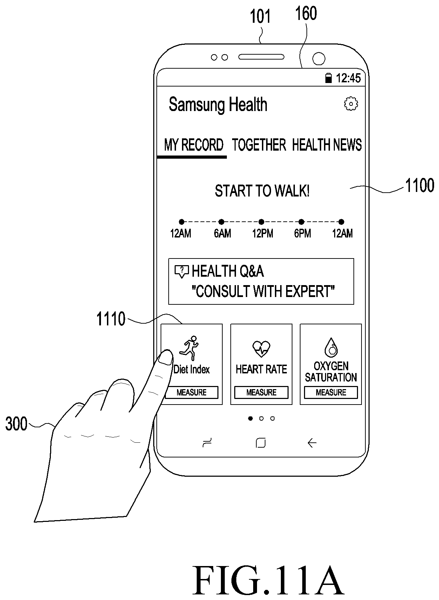

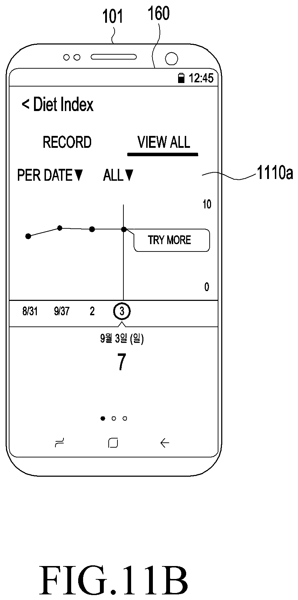

FIGs. 11A and 11B are illustrations of a function/operation for providing comprehensive biometric information based on biometric information, according to an embodiment.

Referring to FIGs. 11A and 11B, the electronic device 101 may provide the user 300 with comprehensive information (e.g., a diet index) regarding a plurality of body components (e.g., fat and water). To provide the user 300 with the comprehensive information, the electronic device 101 may provide information obtained by linearly summing the absorption coefficients calculated (e.g., diet index = absorption coefficient for water + absorption coefficient for fat). The diet index may also be provided as a measured amplitude. The electronic device 101 may provide the comprehensive information to the user 300 based on the sum of absorption coefficients or the amplitude measured. The electronic device 101 may convert the comprehensive information into a range from 0 to 10 and provide the converted data to the user 300. The phrase "absorption coefficient is greater" indicates that the amount of the body component corresponding to the absorption coefficient is greater. Thus, the phrase "linear sum of absorption coefficients is greater" may indicate that the body components (e.g., water and fat) of the user 300 are greater in quantity.

Referring to FIG. 11A, the electronic device 101 may receive an input to identify the comprehensive information from the user 300. The input to identify the comprehensive information may include, e.g., a touch input to a particular interface 1110 on the screen 1100 to provide the user's 300 biometric information. The comprehensive information may include comprehensive information about the user's 300 particular part of the body, e.g., the body water in the skin around the left biceps or the body water in the particular part of the body. The comprehensive information may include comprehensive information about the user's 300 part of the body (e.g., a left arm).

Referring to FIG. 11B, the electronic device 101 may display the comprehensive information, which has been converted into a range from 0 to 10, on the screen 1110a to provide comprehensive information through the display device 160. The electronic device 101 may display history information about the comprehensive information on the screen 1110a.

Light of a wavelength of, e.g., 940nm may be used to provide the comprehensive information. In this case, the electronic device 101 may obtain biometric information about various body components using the 940nm light. In order to provide the comprehensive information, the electronic device 101 may obtain biometric information using a plurality of light beams (e.g., a 940nm light beam and a 975nm light beam) corresponding to designated or selected body component/body components.

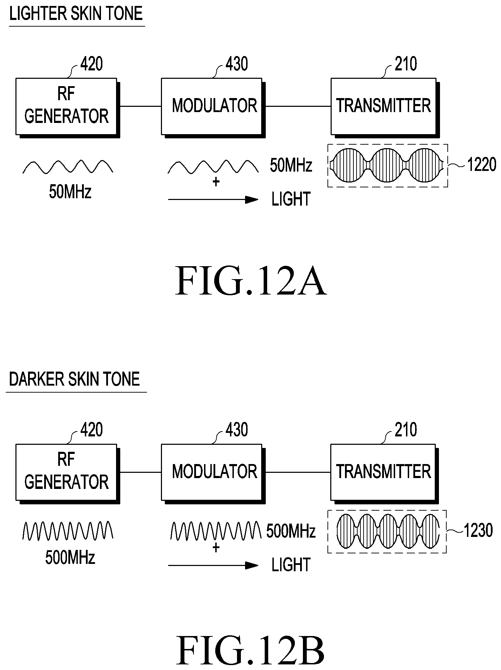

FIGs. 12A and 12B are block diagrams of an electronic device's function/operation for outputting light based on the frequency varied by skin tone that is automatically detected, according to an embodiment.

Referring to FIG. 12A, when skin tone is detected (or set) to be lighter, , the processor 120 may control the RF generator 420, modulator 430, and transmitter 210 to output light 1220 modulated as per a designated frequency. Referring to FIG. 12B, when the skin tone is detected (or set) to be darker, the processor 120 may control the RF generator 420, modulator 430, and transmitter 210 to output light 1230 modulated as per a designated frequency.

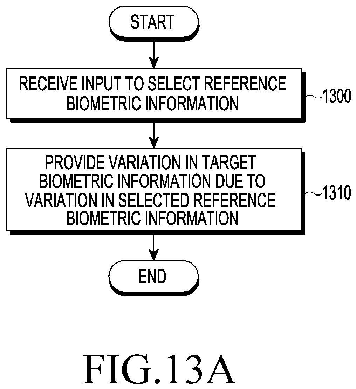

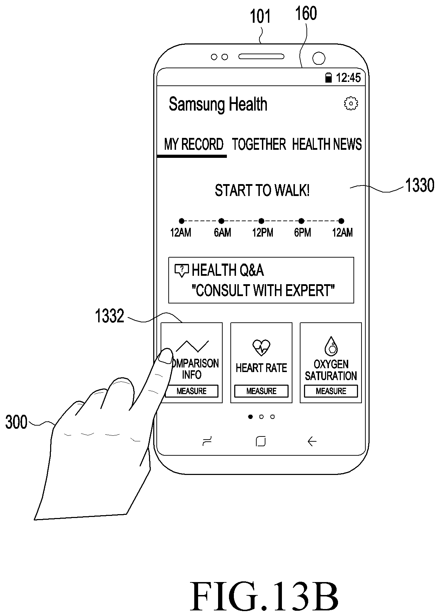

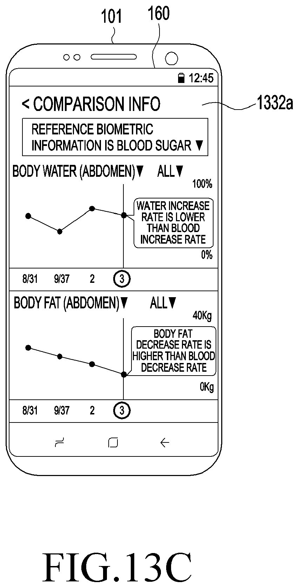

FIGs. 13A, 13B, and 13C are a flowchart and illustrations of an electronic device's 101 function/operation for comparing the variation rate of reference biometric information and the variation rate of target biometric information and providing the results, according to an embodiment.

Referring to FIG. 13A, the electronic device (e.g., the processor 120), in step 1300, may receive an input to select reference biometric information using an input device (e.g., the display device 160).

The electronic device (e.g., the processor 120), in step 1310, may provide variation in target biometric information due to variation in the reference biometric information selected in step 1300, by using a display device 160).

Referring to FIG. 13B, the electronic device 101 may receive an input to identify comparison information from the user 300 on the screen 1330 for comparing the pieces of biometric information. The input to identify comparison information may include, e.g., a touch input on the interface 1332.

FIG. 13C illustrates an example in which blood quantity is set to the reference biometric information. The reference biometric information may be used to provide the user 300 with an indication as to how other biometric information (e.g., water quantity and fat quantity) varies based on the reference biometric information. For example, the electronic device 101 may determine a variation in blood quantity (or reference biometric information) and compare the variation with a variation in the target biometric information. The electronic device 101 may display the results of the comparison on the screen 1332a of the display device 160 (e.g., a display). The results of the comparison may include information regarding the variation in the target biometric information according to the variation in the reference biometric information.

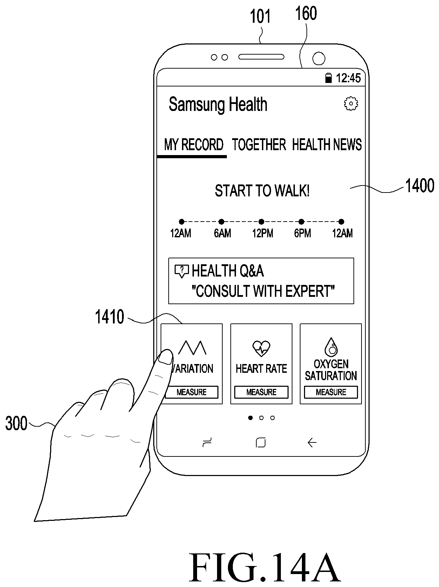

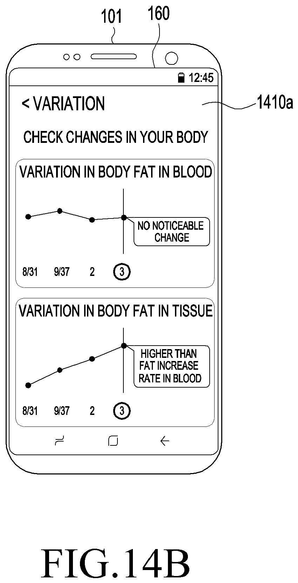

FIGs. 14A and 14B are illustrations of an electronic device's 101 function/operation for separately providing fat quantity in blood and fat quantity in tissue, according to an embodiment.

Referring to FIGs. 14A and 14B, the electronic device 101 may receive an input to identify comparison information about biometric information (e.g., body fat quantity) from the user 300 on the screen 1400 for comparing the pieces of biometric information. The input to identify comparison information may include, e.g., a touch input on the settings interface 1410.

The electronic device 101 may obtain data about the fat quantity in the blood and tissue of the user 300 and display the obtained data on the screen 1410a of the display device 160. The electronic device 101 may provide the user 300 with history information about the obtained biometric information. The electronic device 101 may compare the pieces of information obtained and provide a notification message containing information as per variations in the obtained biometric information. In this case, 940nm-wavelength light may be used.

The electronic device 101 may measure the amount of collagen in the user's 300 particular part of the body (e.g., the forehead) using 910nm-wavelength light or 1020nm-wavelength light. The electronic device 101 may utilize the measured amount of collagen to measure the degree of aging and may recommend appropriate cosmetics depending on the user's 300 degree of aging. The electronic device 101 may also measure the amount of water and may simultaneously measure and provide the amount of water in the skin and the amount of collagen.

The electronic device 101 may measure the amount of sugar in the tissue of the user 300 based on a phase shift, an arrival pattern of photons measured in a histogram, and/or scattering coefficient variations and provide the measurements to the user 300.

The electronic device 101 may indirectly measure (or estimate) increases in the amount of muscle and provide the measurement or estimation to the user 300. The electronic device 101 may determine that the amount of muscle increases when the user's 300 amount of blood increases. Thus, the electronic device 101 may provide the user 300 with the increase rate or decrease rate of the amount of muscle in the user's 300 part of the body measured based on the amount of blood or variation in the amount of blood measured using, e.g., 800nm-wavelength light.

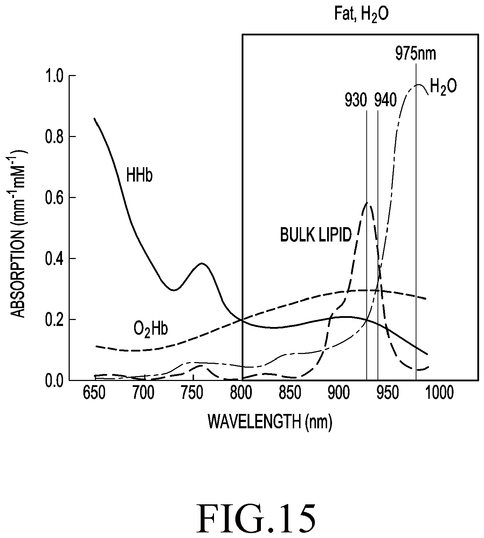

FIG. 15 is a graph of an absorption spectrum for a body composition according to an embodiment.

Referring to FIG. 15, a wavelength of light to obtain biometric information of a user 300 may be determined based on the absorption spectrum of body composition as shown in FIG. 15. For example, when body water is subject to measurement, light of a wavelength near 975nm, which exhibits a relatively good absorption rate for water, may be used. For example, when body fat is subject to measurement, light of a wavelength near 930nm, which exhibits a relatively better absorption rate for fat than other wavelengths, may be used.

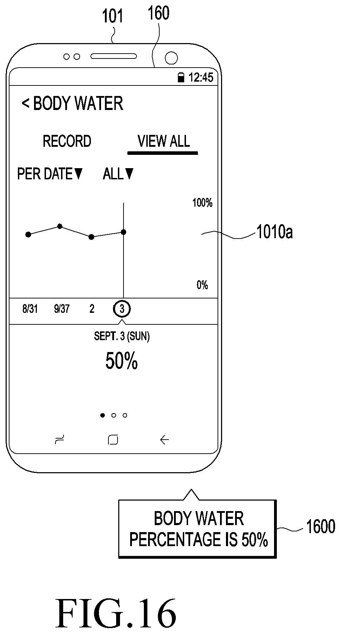

FIG. 16 is a illustration of a function/operation for providing a user 300 with biometric information obtained by an electronic device 101 via a sound output device, according to an embodiment.

Referring to FIG. 16, the electronic device 101 may output information about biometric information (e.g., body water) obtained (or identified) through a sound output device 155. When the amount of water (e.g., the amount of water in skin) is identified to be 50%, the electronic device 101 may output a sound 1600 (e.g., a voice) speaking, "body water is 50%," using the sound output device 155. The electronic device 101 may display a screen 1010a including the obtained biometric information on the display device 160.

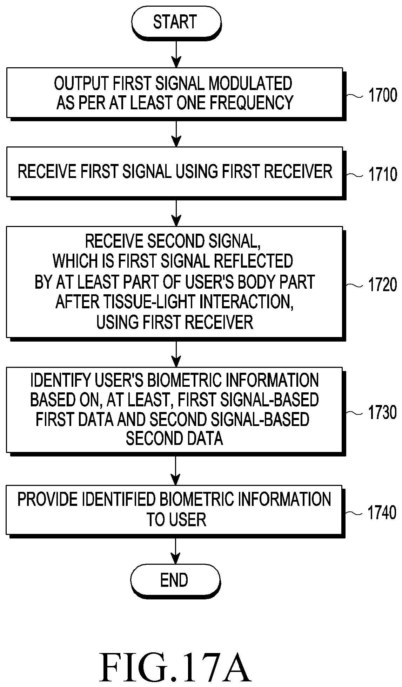

FIG. 17A is a flowchart of a method for operating an electronic device 101, according to an embodiment.

Referring to FIG. 17A, the electronic device (e.g., the processor 120), in step 1700, may output a first signal, which has been modulated as per at least one frequency, using a transmitter 210.

The electronic device (e.g., the processor 120), in step 1710, may receive the first signal using a first receiver 222 (e.g., the first light receiving device DET 1).

The electronic device (e.g., the processor 120), in step 1720, may receive a second signal, which is the first signal reflected by at least a part of the user's body after a tissue-light interaction, using a second receiver 224 (e.g., the second light receiving device DET 2, the third light receiving device DET 3, and/or the fourth light receiving device DET 4). Steps 1710 and 1720 may be performed simultaneously or sequentially.

The electronic device (e.g., the processor 120), in step 1730, may identify the biometric information of the user 300 based on, at least, first signal-based first data and second signal-based second data.

The electronic device (e.g., the processor 120), in step 1740, may provide the identified biometric information to the user.

The biometric information obtained may be shared with an external electronic device(s) through a communication module 190.

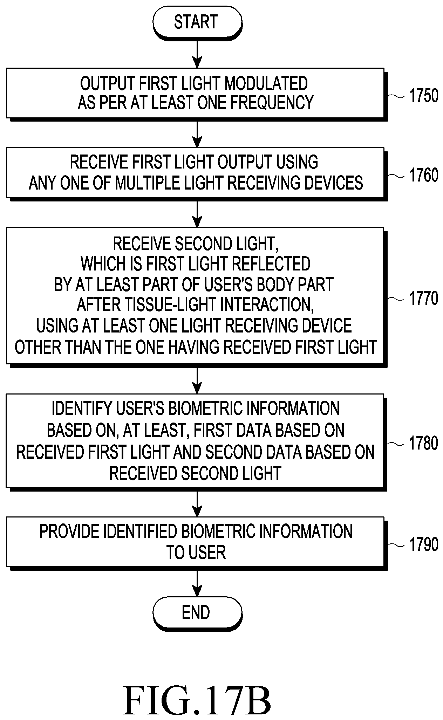

Referring to FIG. 17B, according to an embodiment, the electronic device (e.g., the processor 120), in step 1750, may output first light 310, which has been modulated as per at least one frequency, using a light emitting device.

The electronic device (e.g., the processor 120), in step 1760, may receive the output first light using any one (e.g., the first light receiving device DET 1) of a plurality of light receiving devices.

The electronic device (e.g., the processor 120), in step 1770, may receive second light 330, which is the first light reflected by at least part of the user's body after a tissue-light interaction, using at least one light receiving device (e.g., the second light receiving device DET 2, the third light receiving device DET 3, or the fourth light receiving device DET 4) of the other light receiving devices other than the light receiving device having received the first light or an additional light receiving device.

The electronic device (e.g., the processor 120), in step 1780, may identify the user's biometric information based on, at least, the first data which is based on the received first light and the second data which is based on the received second data.

The electronic device (e.g., the processor 120), in step 1790, may provide the identified biometric information to the user.

Various embodiments of the present disclosure may apply likewise when the sensor module 200 uses a time-of-flight (ToF) sensor. In this case, the ToF sensor may include at least two light sources emitting different wavelengths of light. The ToF sensor may include at least two ultrasound output modules emitting (or producing) different frequencies of ultrasounds.

Heart rate, oxygen saturation, and stress index may also be measured according to various embodiments of the present disclosure. For example, when the sensor includes light sources with wavelengths of 66nm, 880nm, 930nm, and 975nm, heart rate, oxygen saturation, stress index, and fat (or water) may be measured by the single sensor module (e.g., an optical sensor module). However, the wavelengths, 66nm, 880nm, 930nm, and 975nm are exemplary, and other various wavelengths may be used to measure various pieces of biometric information including heart rate, oxygen saturation, stress index, and fat (and water).

According to an embodiment, upon measuring heart rate, the electronic device 101 may set the modulation frequency to 0Hz. However, the modulation frequency, 0Hz, is merely an example, and other various modulation frequencies may be used to measure heart rate.

According to an embodiment, the biometric information may be automatically obtained at preset cycles (or without any input from the user 300 and may be provided to the user at the cycles.

According to an embodiment, an electronic device may include a sensor module including a transmitter, a first receiver, and a second receiver and a processor operatively connected with the sensor module, wherein the processor may be configured to output a first signal modulated as per at least one frequency using the transmitter, receive the first signal using the first receiver, receive a second signal using the second receiver, the second signal being the first signal reflected by at least a part of a user's body after a tissue-light interaction, identify the user's biometric information based on, at least, first data and second data, the first data based on the first signal and the second data based on the second signal, and provide the biometric information to the user.

The sensor module includes a first area and a second area spatially separated by a rib included in a housing of the sensor module, the rib being lower in height than the housing. The transmitter is disposed in the first area, and the first receiver and the second receiver are disposed in the second area. The sensor module may include an optical guide provided between the first area and the second area and configured to allow the first receiver to receive the first signal from the transmitter.

The first receiver and the second receiver may be configured in a single array. The single array includes a linear array.

The sensor module includes a first area and a second area. According to an example not falling under the scope of the claims, the transmitter and the first receiver positioned adjacent to the transmitter may be disposed in the first area, and the second receiver may be disposed in the second area.

The electronic device may further include an optical structure provided at, at least, part of an upper portion of the transmitter, the first receiver, and the second receiver and configured to refract the first signal and the second signal to change the path of the first signal and the second signal.

The electronic device may further include an RF generator and a modulator. The processor may be configured to generate the at least one frequency using the RF generator and modulate the first signal into the at least one frequency using the modulator.

The processor may be configured to set at least one amplitude for the first signal to be output from the transmitter and sequentially output first light using the transmitter, according to the at least one amplitude.

The processor may be configured to compare the amplitude and phase of the first signal based on the first data with the amplitude and phase of the second signal based on the second data, determine a variation in amplitude and phase of the first light, and obtain the user's biometric information based on the comparison of amplitude and phase.

The processor may be configured to calculate an absorption coefficient and a scattering coefficient based on the comparison of amplitude and phase or the arrival pattern of photons measured in the histogram and to obtain the biometric information based on at least part of the absorption coefficient and scattering coefficient calculated.

The processor may be configured to display a notification message for the biometric information using a display of the electronic device and output a notification for the biometric information using a sound output device.

The transmitter may include a ToF light source. The processor may be configured to obtain information regarding the user's glucose or blood sugar based on light emitted from the ToF light source.

The processor may be configured to generate a histogram including information about a receiving pattern of the second signal and obtain the biometric information based on the generated histogram.

According to an example not falling under the scope of the claims, a method for operating an electronic device may include outputting a first signal modulated based on at least one frequency using a transmitter of the electronic device, receiving the first signal using a first receiver of the electronic device, receiving a second signal using a second receiver of the electronic device, the second signal being the first signal reflected by at least part of a user's body part after a tissue-light interaction, identifying the user's biometric information based on, at least, first data and second data, the first data based on the first signal and the second data based on the second signal, and providing the biometric information to the user.

According to an embodiment, an electronic device may include a display, a sensor module including at least one light emitting device, a plurality of light receiving devices, and an optical guide configured to guide at least part of light emitted from the light emitting device to a path, and a processor operatively connected with the display and the sensor module, wherein the processor may be configured to output first light modulated as per at least one frequency using the light emitting device, receive the first light using any one of the plurality of light receiving devices, the first light guided to the any one light receiving device by the optical guide, receive second light using at least one of the other light receiving devices other than the any one light receiving device receiving the first light, the second light being the first light reflected by at least part of a user's body after a tissue-light interaction, identify the user's biometric information based on, at least, first data and second data, the first data based on the received first light and the second data based on the received second light, and provide the user with the biometric information using the display, and wherein the any one light receiving device and the at least one light receiving device may be configured in a single sensor array or may be disposed in different areas spatially separated by a rib in the sensor module.

The electronic device is, according to the invention, a wearable electronic device, and according to various examples not falling under the scope of the claims may be one of various types of electronic devices. The electronic devices may include, for example, a portable communication device (e.g., a smart phone), a computer device, a portable multimedia device, a portable medical device, a camera, or a home appliance. According to an embodiment of the disclosure not falling under the scope of the claims, the electronic device is not limited to the above-listed embodiments. With regard to the description of the drawings, similar reference numerals may be used to refer to similar or related elements. It is to be understood that a singular form of a noun corresponding to an item may include one or more of the things, unless the relevant context clearly indicates otherwise. As used herein, each of such phrases as "A or B," "at least one of A and B," "at least one of A or B," "A, B, or C," "at least one of A, B, and C," and "at least one of A, B, or C," may include all possible combinations of the items enumerated together in a corresponding one of the phrases. As used herein, such terms as "1st" and "2nd," or "first" and "second" may be used to simply distinguish a corresponding component from another, and does not limit the components in other aspect (e.g., importance or order). It is to be understood that if an element (e.g., a first element) is referred to, with or without the term "operatively" or "communicatively", as "coupled with," "coupled to," "connected with," or "connected to" another element (e.g., a second element), it means that the element may be coupled with the other element directly (e.g., wiredly), wirelessly, or via a third element.

As used herein, the term "module" may include a unit implemented in hardware, software, or firmware, and may interchangeably be used with other terms, for example, "logic," "logic block," "part," or "circuitry". A module may be a single integral component, or a minimum unit or part thereof, adapted to perform one or more functions. For example, according to an embodiment, the module may be implemented in a form of an application-specific integrated circuit (ASIC).

Various embodiments as set forth herein may be implemented as software (e.g., the program 140) including one or more instructions that are stored in a storage medium (e.g., internal memory 136 or external memory 138) that is readable by a machine (e.g., the electronic device 101); however, the software does not fall under the scope of the claims by itself. For example, a processor (e.g., the processor 120) of the machine (e.g., the electronic device 101) may invoke at least one of the one or more instructions stored in the storage medium, and execute it, with or without using one or more other components under the control of the processor. This allows the machine to be operated to perform at least one function according to the at least one instruction invoked. The one or more instructions may include a code generated by a complier or a code executable by an interpreter. The machine-readable storage medium may be provided in the form of a non-transitory storage medium. Wherein, the term "non-transitory" simply means that the storage medium is a tangible device, and does not include a signal (e.g., an electromagnetic wave), but this term does not differentiate between where data is semi-permanently stored in the storage medium and where the data is temporarily stored in the storage medium.

According to an example not falling under the scope of the claims, a method according to various embodiments of the disclosure may be included and provided in a computer program product. The computer program products may be traded as commodities between sellers and buyers. The computer program product may be distributed in the form of a machine-readable storage medium (e.g., compact disc read only memory (CD-ROM)), or be distributed (e.g., downloaded or uploaded) online via an application store (e.g., Play Store™), or between two user devices (e.g., smart phones) directly. If distributed online, at least part of the computer program product may be temporarily generated or at least temporarily stored in the machine-readable storage medium, such as memory of the manufacturer's server, a server of the application store, or a relay server.

According to various embodiments, each component (e.g., a module or a program) of the above-described components may include a single entity or multiple entities. According to various embodiments, one or more of the above-described components may be omitted, or one or more other components may be added. Alternatively or additionally, a plurality of components (e.g., modules or programs) may be integrated into a single component. In such a case, according to various embodiments, the integrated component may still perform one or more functions of each of the plurality of components in the same or similar manner as they are performed by a corresponding one of the plurality of components before the integration. According to various embodiments, operations performed by the module, the program, or another component may be carried out sequentially, in parallel, repeatedly, or heuristically, or one or more of the operations may be executed in a different order or omitted, or one or more other operations may be added.

As is apparent from the foregoing description, various embodiments of the disclosure may provide the user with biometric information, such as glucose, water, or fat, in which the user would be more interested in her daily life, using modulated light.

Various embodiments of the disclosure may provide the user with the resultant information that is obtained by comparing and analyzing pieces of biometric information obtained, enabling a comprehensive checkup on the user's current body condition.

**Claims**

1.   A wearable electronic device, comprising:

a sensor module (200) including a transmitter (210), a first receiver (222), and a second receiver (224);
a processor operatively connected with the sensor module (200), wherein the processor is configured to output out of the wearable electronic device a first signal (310) of one type from a group comprising at least light and ultrasound using the transmitter, receive a reference part (320) of the first signal using the first receiver, receive a second signal (330) using the second receiver, wherein the second signal is another part of the first signal reflected by a part of a user's body (300) after a tissue-signal interaction within the part of the user's body, identify the user's biometric information based on, at least, first data and second data, wherein the first data is based on the first signal and the second data is based on the second signal;
a display (160),
a structure (342a) provided at an upper portion of at least the transmitter and the second receiver and configured to redirect the other part of the first signal to the user, and to redirect the second signal reflected from the user to the second receiver; and
wherein the sensor module (200) further includes a first area (202) and a second area (204) spatially separated by a rib (240) included in a housing (230) of the sensor module, wherein the rib is lower in height than the housing, and wherein the transmitter (210) is disposed in the first area, and the first receiver (222) and the second receiver (224) are disposed in the second area, and wherein the sensor module (200) further includes a guide, wherein the guide (206) is configured to extend between the first area and the second area over the rib (240), wherein a portion of the guide (206) is in contact with any one of the first receiver (222) and the second receiver (224), to allow the first receiver (222) to receive the reference part (320) of the first signal from the transmitter,
wherein the display (160) is configured to present the biometric information to the user (300).

2. The wearable electronic device of claim 1, wherein the first receiver and the second receiver are configured in a single array (222 + 224).

3. The wearable electronic device of claim 1, wherein the structure (342a) provided at the upper portion of at least the transmitter and the second receiver comprises a total internal reflection, TIR, Fresnel lens configured to refract the first signal and the second signal to change a path of the first signal and the second signal.

**Patentansprüche**

1. Am Körper tragbare elektronische Vorrichtung, die Folgendes umfasst:

ein Sensormodul (200) einschließlich einer Übertragungseinrichtung (210), eines ersten Empfängers (222) und eines zweiten Empfängers (224);
einen Prozessor, der mit dem Sensormodul (200) operativ verbunden ist, wobei der Prozessor zu Folgendem konfiguriert ist: Ausgeben eines ersten Signals (310) eines Typs aus einer Gruppe, die zumindest Licht und Ultraschall umfasst, aus der tragbaren elektronischen Vorrichtung unter Verwendung des Senders, Empfangen eines Referenzteils (320) des ersten Signals unter Verwendung des ersten Empfängers, Empfangen eines zweiten Signals (330) unter Verwendung des zweiten Empfängers, wobei das zweite Signal ein anderer Teil des ersten Signals ist, das von einem Teil des Körpers eines Benutzers (300) reflektiert wird, nachdem eine Gewebe-Signal-Interaktion innerhalb des Körperteils des Benutzers erfolgt ist, Identifizieren von biometrischen Informationen des Benutzers zumindest basierend auf ersten Daten und zweiten Daten, wobei die ersten Daten auf dem ersten Signal basieren und die zweiten Daten auf dem zweiten Signal basieren;
eine Anzeige (160);
eine Struktur (342a), die an einem oberen Abschnitt von zumindest dem Sender und dem zweiten Empfänger vorgesehen ist und konfiguriert ist, um den anderen Teil des ersten Signals zu dem Benutzer umzuleiten, und um das zweite Signal, das von dem Benutzer reflektiert wird, zu dem zweiten Empfänger umzuleiten; und wobei das Sensormodul (200) ferner einen ersten Bereich (202) und einen zweiten Bereich (204) umfasst, die durch eine in einem Gehäuse (230) des Sensormoduls enthaltene Rippe (240) räumlich getrennt sind, wobei die Rippe eine geringere Höhe aufweist als das Gehäuse, und wobei der Sender (210) im ersten Bereich angeordnet ist, und der erste Empfänger (222) und der zweite Empfänger (224) im zweiten Bereich angeordnet sind, und wobei das Sensormodul (200) ferner eine Führung enthält, wobei die Führung (206) so konfiguriert ist, dass sie sich zwischen dem ersten Bereich und dem zweiten Bereich über die Rippe (240) erstreckt, wobei ein Teil der Führung (206) mit einem beliebigen aus dem ersten Empfänger (222) und dem zweiten Empfänger (224) in Kontakt steht, damit der erste Empfänger (222) den Referenzteil (320) des ersten Signals von dem Sender empfangen kann,
wobei die Anzeige (160) so konfiguriert ist, dass sie dem Benutzer (300) die biometrischen Informationen präsentiert.

2. Am Körper tragbare elektronische Vorrichtung nach Anspruch 1, wobei der erste Empfänger und der zweite Empfänger in einem einzigen Array (222 + 224) konfiguriert sind.

3. Am Körper tragbare elektronische Vorrichtung nach Anspruch 1, wobei die Struktur (342a), die an dem oberen Abschnitt zumindest des Senders und des zweiten Empfängers vorgesehen ist, eine TIR-Fresnel-Linse (Total Internal Reflection) umfasst, die so konfiguriert ist, dass sie das erste Signal und das zweite Signal refraktiert, um einen Pfad des ersten und des zweiten Signals zu ändern.

**Revendications**

1. Objet personnel connecté, comprenant :

un module de détection (200) comportant un émetteur (210), un premier récepteur (222) et un deuxième récepteur (224) ;
un processeur relié de manière fonctionnelle au module de détection (200), ledit processeur étant conçu pour faire émettre par l'objet personnel connecté un premier signal (310) d'un type appartenant à un groupe comprenant au moins les signaux lumineux et ultrasonores au moyen de l'émetteur, pour recevoir une partie de référence (320) du premier signal au moyen du premier récepteur, pour recevoir un deuxième signal (330) au moyen du deuxième récepteur, ledit deuxième signal consistant en une autre partie du premier signal réfléchie par une partie du corps d'un utilisateur (300) après une interaction tissu-signal au sein de ladite partie du corps de l'utilisateur, pour identifier des informations biométriques de l'utilisateur compte tenu, au moins, de premières données et de deuxièmes données, lesdites premières données reposant sur le premier signal et lesdites deuxièmes données reposant sur le deuxième signal ;

un écran (160) ; et

une structure (342a) prévue au niveau d'une partie supérieure au moins de l'émetteur et du deuxième récepteur et conçue pour rediriger l'autre partie du premier signal vers l'utilisateur, et pour rediriger le deuxième signal réfléchi par l'utilisateur vers le deuxième récepteur ;

ledit module de détection (200) comportant en outre une première zone (202) et une deuxième zone (204) espacées spatialement par une nervure (240) présente dans un boîtier (230) du module de détection, ladite nervure étant de moindre hauteur que le boîtier, et ledit émetteur (210) étant disposé dans la première zone, et le premier récepteur (222) et le deuxième récepteur (224) étant disposés dans la deuxième zone, et ledit module de détection (200) comportant en outre un guide, ledit guide (206) étant conçu pour s'étendre entre la première zone et la deuxième zone par-dessus la nervure (240), une portion du guide (206) étant en contact avec le premier récepteur (222) ou le deuxième récepteur (224), pour permettre au premier récepteur (222) de recevoir la partie de référence (320) du premier signal en provenance de l'émetteur,

ledit écran (160) étant conçu pour présenter les informations biométriques à l'utilisateur (300).

2. Objet personnel connecté selon la revendication 1, dans lequel le premier récepteur et le deuxième récepteur sont conçus dans un même ensemble (222 + 224).

3. Objet personnel connecté selon la revendication 1, dans lequel la structure (342a) prévue au niveau de la partie supérieure au moins de l'émetteur et du deuxième récepteur comprend une lentille de Fresnel à réflexion interne totale conçue pour réfracter le premier signal et le deuxième signal pour changer la trajectoire du premier signal et du deuxième signal.

FIG.1

ELECTRONIC DEVICE 101

INPUT DEVICE 150

SOUND OUTPUT DEVICE 155

DISPLAY DEVICE 160

MEMORY 130

VOLATILE MEMORY 132

NON-VOLATILE MEMORY 134

INTERNAL MEMORY 136

EXTERNAL MEMORY 138

BATTERY 189

PROCESSOR 120

MAIN PROCESSOR 121

AUXILIARY PROCESSOR 123

POWER MANAGEMENT MODULE 188

COMMUNICATION MODULE 190

WIRELESS COMMUNICATION MODULE 192

WIRED COMMUNICATION MODULE 194

SUBSCRIBER IDENTIFICATION MODULE 196

ANTENNA MODULE 197

AUDIO MODULE 170

SENSOR MODULE 176

INTERFACE 177

CONNECTION TERMINAL 178

HAPTIC MODULE 179

CAMERA MODULE 180

PROGRAM 140

APPLICATION 146

MIDDLEWARE 144

OPERATING SYSTEM 142

100

NETWORK 199

ELECTRONIC DEVICE 104

ELECTRONIC DEVICE 102

SERVER 108

198

EP 3 527 124 B1

FIG.2A

FIG.2B

23

FIG.3A

FIG.3B

FIG.3C

FIG.3D

FIG.3E

*101*

*120* — PROCESSOR

*410* — AFE

*420* — RF GENERATOR

*430* — MODULATOR

*200*

*210* — TRANSMITTER

*460* — ADC

*450* — LOW PASS FILTER

*440* — I/Q DEMODULATOR

*220*

DET1 — *222*
DET2
DET3 — *224*
DET4

# FIG.4A

*420* — RF GENERATOR

*430* — MODULATOR

*210* — TRANSMITTER

100MHz

100MHz + LIGHT

*470*

# FIG.4B

FIG.5A

FIG.5B

FIG.5C

START

RECEIVE REQUEST TO PROVIDE
USER'S BIOMETRIC INFORMATION — 600

OUTPUT FIRST SIGNAL TO OBTAIN
BIOMETRIC INFORMATION — 610

RECEIVE SECOND SIGNAL CORRESPONDING
TO FIRST SIGNAL OUTPUT — 620

COMPARE AMPLITUDE AND
PHASE OF RECEIVED SECOND SIGNAL WITH
AMPLITUDE AND PHASE OF FIRST SIGNAL — 630

IDENTIFY USER'S BIOMETRIC INFORMATION
BASED ON RESULT OF COMPARISON — 640

PROVIDE IDENTIFIED BIOMETRIC
INFORMATION TO USER — 650

END

# FIG.6A

# FIG.6B

# FIG.6C

START

STORE FIRST BIOMETRIC INFORMATION — 700

RECEIVE REQUEST TO PROVIDE
SECOND BIOMETRIC INFORMATION — 710

OUTPUT FIRST SIGNAL TO OBTAIN
SECOND BIOMETRIC INFORMATION — 720

RECEIVE SECOND SIGNAL CORRESPONDING
TO FIRST SIGNAL OUTPUT — 730

COMPARE AMPLITUDE AND
PHASE OF RECEIVED SECOND SIGNAL WITH
AMPLITUDE AND PHASE OF FIRST SIGNAL — 740

IDENTIFY SECOND BIOMETRIC INFORMATION
BASED ON RESULT OF COMPARISON — 750

PROVIDE HISTORY INFORMATION ABOUT
BIOMETRIC INFORMATION BASED ON
FIRST BIOMETRIC INFORMATION AND
SECOND BIOMETRIC INFORMATION — 760

END

FIG.7A

FIG.7B

FIG.7C

START

RECEIVE REQUEST TO PROVIDE
BIOMETRIC INFORMATION — 800

OUTPUT AT LEAST TWO OR MORE ⌐805
FIRST SIGNALS MODULATED AS PER
AT LEAST TWO OR MORE FREQUENCIES

RECEIVE AT LEAST TWO OR MORE ⌐810
FIRST SIGNALS OUTPUT USING
FIRST RECEIVER

RECEIVE AT LEAST TWO OR MORE ⌐815
SECOND SIGNALS CORRESPONDING TO
AT LEAST TWO OR MORE FIRST SIGNALS
OUTPUT USING SECOND RECEIVER

DETERMINE AMPLITUDE ATTENUATION AND ⌐820
PHASE SHIFT BASED ON FIRST SIGNALS
AND SECOND SIGNALS RECEIVED

CALCULATE ABSORPTION COEFFICIENT ⌐825
AND SCATTERING COEFFICIENT
BASED ON DETERMINATION

OBTAIN BIOMETRIC INFORMATION ⌐830
BASED ON ABSORPTION COEFFICIENT AND
SCATTERING COEFFICIENT CALCULATED

PROVIDE OBTAINED BIOMETRIC ⌐835
INFORMATION

END

FIG.8A

START

RECEIVE REQUEST TO PROVIDE
BIOMETRIC INFORMATION — 840

OUTPUT FIRST SIGNAL MODULATED
AS PER ONE FREQUENCY — 845

RECEIVE FIRST SIGNAL OUTPUT
USING FIRST RECEIVER — 850

RECEIVE SECOND SIGNAL CORRESPONING
TO FIRST SIGNAL OUTPUT
USING SECOND RECEIVER — 855

DETERMINE AMPLITUDE ATTENUATION AND
PHASE SHIFT BASED ON FIRST SIGNAL
AND SECOND SIGNAL RECEIVED — 860

CALCULATE ABSORPTION COEFFICIENT
AND SCATTERING COEFFICIENT
BASED ON DETERMINATION — 865

OBTAIN BIOMETRIC INFORMATION
BASED ON ABSORPTION COEFFICIENT AND
SCATTERING COEFFICIENT CALCULATED — 870

PROVIDE OBTAINED BIOMETRIC
INFORMATION — 875

END

FIG.8B

# FIG.9A

# FIG.9B

FIG.9C

101    160

☐ 12:45

< VARIATION IN BODY FAT (ARM)

RECORD        VIEW ALL

PER DATE▼    ALL▼

30㎜

912b

0㎜

8/31    9/37    2    ③

SEPT. 3 (SUN)

5mm

● ○ ○

FIG.9D

FIG.9E

101

900

# FIG.9F

FIG.9G

930

SEPT. 3 (SUN)

GLUCOSE VARIED BY

+5%

920

FIG.9H

FIG.10A

101    160

< VARIATION IN BODY WATER

RECORD        VIEW ALL

PER DATE▼    ALL▼

100%

1010a

0%

8/31    9/37    2    ③

SEPT. 3 (SUN)

5% UP

FIG.10B

FIG.11A

FIG.11B

LIGHTER SKIN TONE

420
RF GENERATOR

430
MODULATOR

210
TRANSMITTER

50MHz

50MHz
+
LIGHT

~1220

# FIG.12A

DARKER SKIN TONE

420
RF GENERATOR

430
MODULATOR

210
TRANSMITTER

500MHz

500MHz
+
LIGHT

~1230

# FIG.12B

START

RECEIVE INPUT TO SELECT REFERENCE
BIOMETRIC INFORMATION ~1300

PROVIDE VARIATION IN TARGET
BIOMETRIC INFORMATION DUE TO
VARIATION IN SELECTED REFERENCE
BIOMETRIC INFORMATION ~1310

END

# FIG.13A

FIG.13B

**FIG.13C**

FIG.14A

FIG.14B

FIG.15

FIG.16

START

OUTPUT FIRST SIGNAL MODULATED
AS PER AT LEAST ONE FREQUENCY    ～ 1700

RECEIVE FIRST SIGNAL USING FIRST RECEIVER    ～ 1710

RECEIVE SECOND SIGNAL,
WHICH IS FIRST SIGNAL REFLECTED
BY AT LEAST PART OF USER'S BODY PART    ～ 1720
AFTER TISSUE-LIGHT INTERACTION,
USING FIRST RECEIVER

IDENTIFY USER'S BIOMETRIC INFORMATION
BASED ON, AT LEAST, FIRST SIGNAL-BASED
FIRST DATA AND SECOND SIGNAL-BASED    ～ 1730
SECOND DATA

PROVIDE IDENTIFIED BIOMETRIC INFORMATION
TO USER    ～ 1740

END

# FIG.17A

START

OUTPUT FIRST LIGHT MODULATED
AS PER AT LEAST ONE FREQUENCY  — 1750

RECEIVE FIRST LIGHT OUTPUT USING
ANY ONE OF MULTIPLE LIGHT RECEIVING DEVICES  — 1760

RECEIVE SECOND LIGHT,
WHICH IS FIRST LIGHT REFLECTED
BY AT LEAST PART OF USER'S BODY PART
AFTER TISSUE-LIGHT INTERACTION,
USING AT LEAST ONE LIGHT RECEIVING DEVICE
OTHER THAN THE ONE HAVING RECEIVED FIRST LIGHT  — 1770

IDENTIFY USER'S BIOMETRIC INFORMATION
BASED ON, AT LEAST, FIRST DATA BASED ON
RECEIVED FIRST LIGHT AND SECOND DATA BASED ON
RECEIVED SECOND LIGHT  — 1780

PROVIDE IDENTIFIED BIOMETRIC INFORMATION
TO USER  — 1790

END

# FIG.17B

**REFERENCES CITED IN THE DESCRIPTION**

**Patent documents cited in the description**

- WO 2017027551 A